# EUROPEAN PATENT APPLICATION

(11) **EP 4 650 437 A1**
(43) Date of publication of application: **19.11.2025**
(21) Application number: 24382526.2
(22) Date of filing: 16.05.2024
(51) Int. Cl.: C12N 5/00, B33Y 70/00, B33Y 80/00, C12N 5/077, C12N 5/09, G01N 33/50, B33Y 10/00

(54) **TUMOR MODEL AND PROCESS OF PREPARATION THEREOF**

(71) Applicant: Asociación Centro de Investigación en Biomateriales - CIC biomaGune, 20014 San Sebastián (ES)
(72) Inventor: GONZÁLEZ CALLEJO, Patricia, 20014 San Sebastián (Gipuzkoa) (ES); GARCÍA ASTRAIN, Clara María, 20014 San Sebastián (Gipuzkoa) (ES); LIZ MARZÁN, Luis Manuel, 20014 San Sebastián (Gipuzkoa) (ES)
(74) Representative: Herrero & Asociados, S.L.

(57) **Abstract**

The present invention refers to a process for preparing a three-dimensional tumor model having a core-shell structure using bioprinting techniques leading to tumor models capable of better reproducing the physiopathological complexity of real tumors, mimicking the tumor microenvironment and the original tumor tissue extracellular matrix. The process is based on the use of dECM (decellularized extracellular matrix) bioinks but with the particularity that no exogenous biopolymers that modify the rheological properties to improve printability are needed. The absence of rheological modifiers in the bioinks avoids any kind of physical or biochemical interference in the tumor model so obtained.

## Description

### FIELD OF THE INVENTION

The present invention belongs to the field of tumor models for drug testing and for *in vitro* research purposes. More specifically, the present invention refers to a process for preparing a three-dimensional tumor model having a core-shell structure using special bioprinting strategies leading to tumor models capable of better reproducing the physiopathological complexity of real tumors, mimicking the tumor microenvironment and the extracellular matrix of the tissue where the tumor originated. The process is based on the use of dECM (decellularized extracellular matrix) bioinks preferably with the particularity that no exogenous biopolymers that modify the rheological properties to improve printability are needed. The absence of rheological modifiers in the bioinks prevents any kind of physical or biochemical interference in the tumor model.

In addition, the invention refers to a three-dimensional tumor model obtained or obtainable by the process of the invention, to methods for testing anticancer drugs based on such tumor model, as well as to the use of the tumor model in developing personalized medicine and developing new therapies based on the understanding of the *in vitro* tumor progression.

### BACKGROUND OF THE INVENTION

Although several advances have been made regarding cancer treatment during the last decades, cancer is still in many cases an incurable disease with a high mortality rate worldwide. Cancer patients display high levels of inter-patient variation in terms of prognosis, response to therapy, and subsequent clinical outcomes. Molecular, phenotypic and functional diversity among tumors from different cancer patients, are features that can dictate therapeutic responses.

Among different cancer subtypes, breast cancer is known to be one of the most heterogeneous, representing the most prevalent disease among women worldwide, the most frequently diagnosed cancer, and the leading cause of cancer mortality in women. In this sense, breast tumors intra-tumoral and inter-tumoral heterogeneity imposes a great variability on patient's prognosis and response to therapy.

For this reason, the development of personalized preclinical studies that guarantee and determine the most appropriate therapeutic options for each patient is of great relevance. The development of a prognostic preclinical *in vitro* model capable of mimicking this diversity *ex vivo* would provide an interesting alternative to identify the most suitable treatment regimens for individual patients in a personalized manner. Unfortunately, there are few *in vitro* tumor models available to reflect tumor complexity that serve to monitor therapeutic effectiveness of pharmacological treatments for each patient. One of the main limitations is the poor reproducibility of the tumor microenvironment (TME). The TME is a highly complex niche where tumor cells are surrounded by various types of non-malignant cells, including stromal and immune cells embedded within the extracellular matrix (ECM), which plays a pivotal role in cancer initiation, progression, and response to treatment. The TME is characterized by the complex bidirectional communication network that is established between cancer cells, stromal cells, and ECM components. Hence, it is essential to preserve the features of the TME when designing *in vitro* tumor models for pre-clinical drug testing. The recreation of the TME entails a challenge when developing complex 3D culture *in vitro* models methods.

Recently, 3D bioprinting has shown great potential in the realistic recreation of tissues, mainly thanks to its ability to assemble various cell types and biomaterials with high spatial resolution, thus forming bioengineered precise 3D constructs that resemble the anatomical structure of tissues in all three dimensions. Thanks to this technology, 3D bioprinted models can recreate the complexity of *in vivo* tumors, including the TME and their functional and structural hierarchies, with high resolution. Different attempts to develop *in vitro* tumor models have been made, such as perfusable layered models (Choi et al., 2015) and spheroid-based metastasis models (Guzman et al., 2017). Other 3D tumor models have also been disclosed in the art (DATTA PALLAB et al: "3D bioprinting for reconstituting the cancer microenvironment" NPJ Precision Oncology, 2020, Vol.4 (1)), but most of them are based in bioink materials and geometries that are far from reproducing the TME.

It should be stressed that, the bioprintable biomaterials (bioinks) must provide suitable micro- and macro-structure, as well as mechanical properties for cell adhesion, proliferation and differentiation in any direction of space. Among the various bioinks of interest used in the above described models, synthetic and natural hydrogels from non-tissue derived sources have been the gold standard materials used for rendering the 3D models.

Tissue-specific decellularized ECM (dECM), obtained by removing all cellular components from various tissue sources, can offer improved TME properties to recreate specific tumor models because they can offer the ability to recreate a specific tumor niche ECM environment, when compared against synthetic and natural hydrogels.

The problem is that dECM-based bioinks usually have to be modified with exogenous biopolymers to improve or allow their printability. For instance, Blanco Fernandez et al. (Appl. Mater. Interfaces, 2022, Vol. 14: 29467-29482) teaches a breast cancer 3D-tumor model in a core-shell structure comprising the use of a dECM-based bioink for bioprinting. However, in order to make the dECM-based bioink bioprintable this document teaches about the need to incorporate gelatin methacrylamide, alginate and type I collagen.

The use of exogenous biopolymers or any other type of rheological modifiers have nevertheless several drawbacks. Introducing materials that are exogenous to the ECM is contrary to the idea of artificially recreating TME. Cells can directly interact with these exogenous materials and influence on cell behavior, evolution and tumor progression. Besides this potential biochemical interference, rheological modifiers are used to increase crosslinking of the bioinks and so they have an impact on cell mobility. The free movement of cells in all spatial dimensions has been proven to be restricted by crosslinking agents. This is especially sensible when the dynamic behavior of cells in metastatic and non-metastatic models is to be analyzed.

Therefore, there is a need to develop 3D tumor models that more faithfully recreate Tumor Microenvironment (TME) and more accurately reproduce the conditions that tumors find in real-life.

Now, the inventors surprisingly found that the use of the so-called FESH bioprinting technology (Freeform Embedding of Suspended Hydrogels) allows to develop a process to prepare a 3D tumor model having an inner core being completely surrounded in all three dimensions by an outer stromal shell exclusively based on bioinks derived from dECM without the need of including any kind of exogenous polymer, rheological modifier or crosslinking agent. The 3D tumor model so obtained is highly and easily reproducible, which renders it a powerful tool in tumor and cancer research, which can be exploited for the development of high throughput drug screening platforms. The 3D model herein disclosed is especially well suited for research of dynamic studies where mobility of cells is a key issue such as in metastatic cancer models.

### BRIEF DESCRIPTION OF THE FIGURES

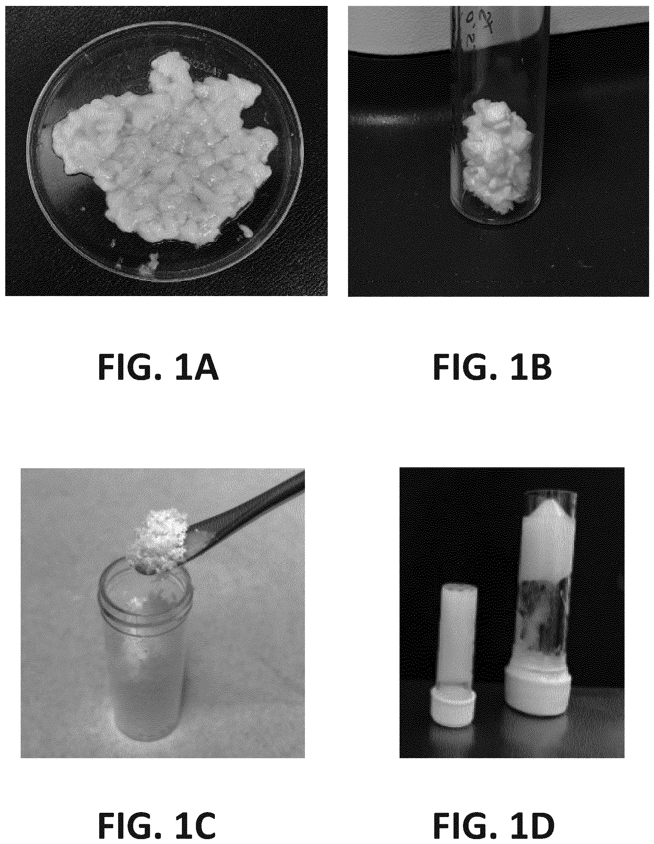

**FIG. 1****: dECM production** Macroscopic images of the obtention of the dissected breast tissue (A), freeze-dried decellularized ECM (B), dECM powder (C) and dECM ink (D).

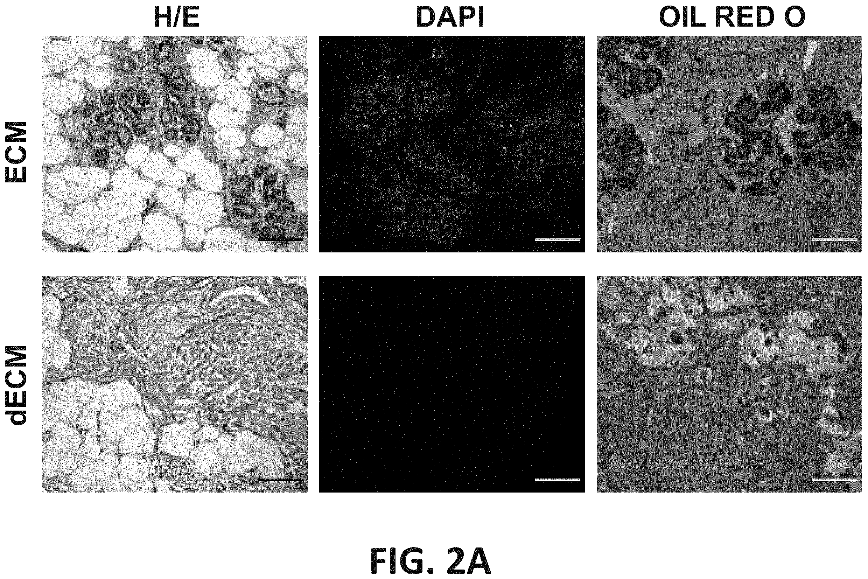

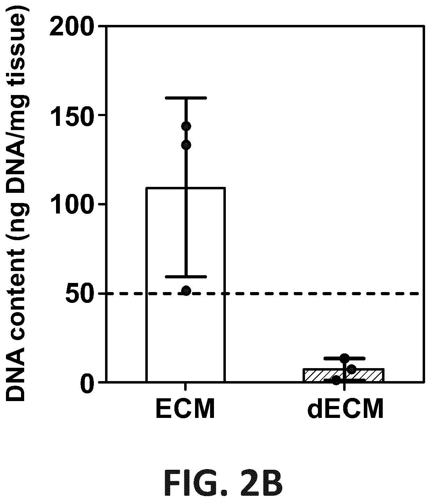

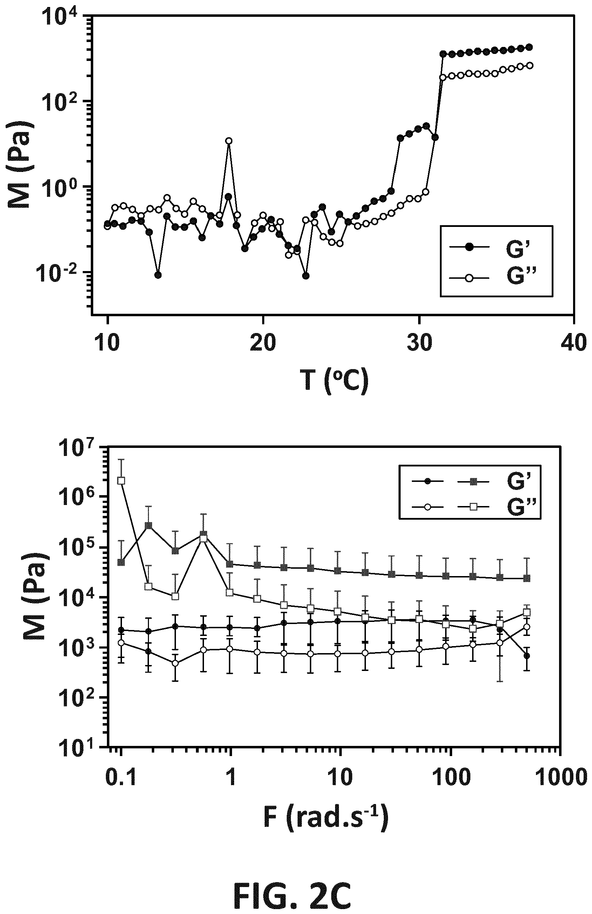

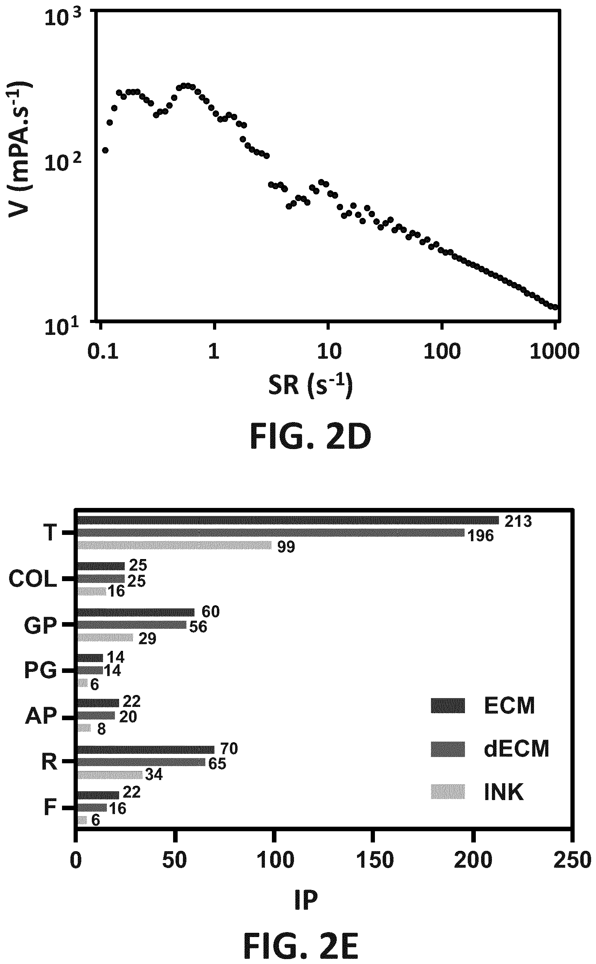

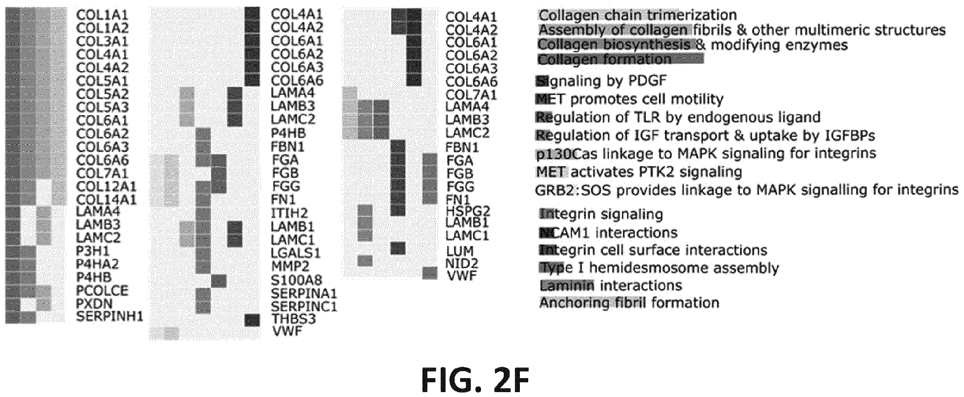

**FIG. 2****: dECM characterization. (A)** Histological analysis of ECM/dECM, showing the tissue structure by H&E staining (left), nuclei by DAPI (center) and delipidation by Oil Red O (OR O, right) staining. Scale bars: 150 µm. **(B)** DNA content of ECM and remnant DNA in dECM after decellularization treatments. Threshold for acceptance criterion set at 50 ng/mg. **(C)** Rheological characterization including temperature ramp and frequency sweep tests of the dECM ink before (black) and after cross-linking (purple). (M: modulus, T: Temperature, F: Frequency) **(D)** Rheological characterization including the flow curve of the dECM ink before cross-linking representing the viscosity (V) versus shear rate (SR), **(E)** Quantification of the number of proteins identified (IP) by LC-MS/MS in the native ECM, dECM and dECM ink. **(F)** Classification by function of the identified proteins within the dECM matrisome (blue, synthesis and assembly of collagenous fibers; pink, cell signaling and regulation; green, ECM organization and cell/ECM interaction molecules).

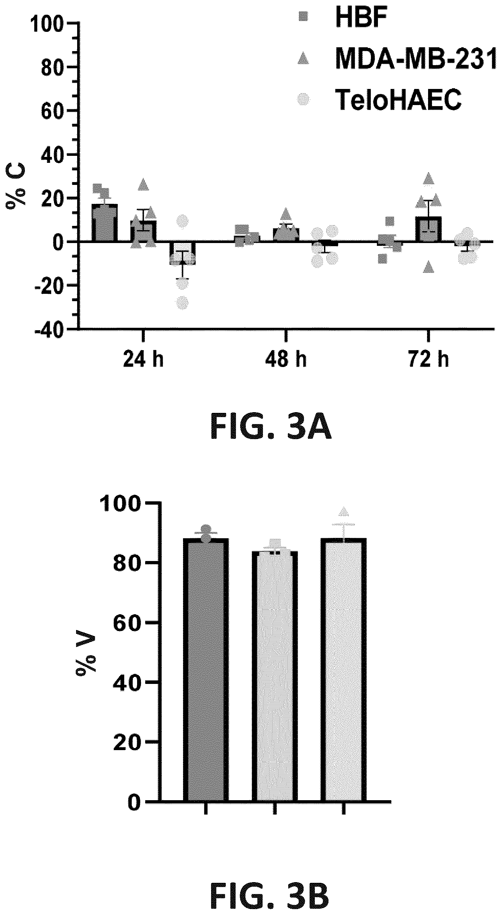

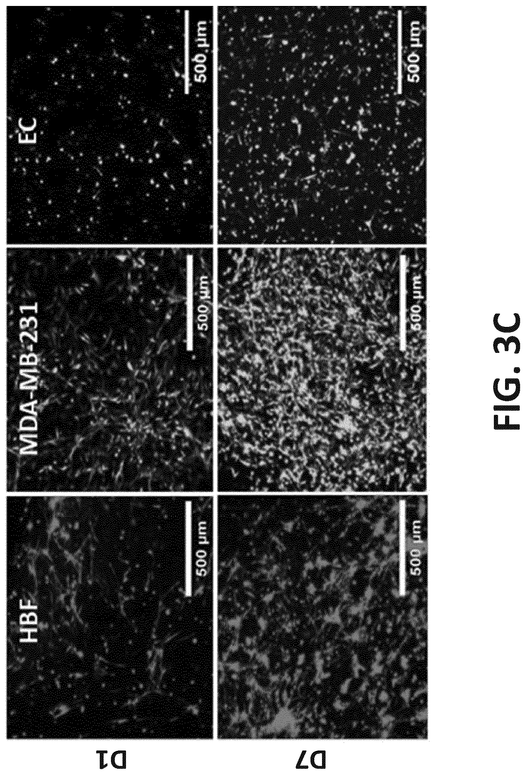

**FIG. 3****: Evaluation of dECM bioink biocompatibility. (A)** Bar plot representing cell cytotoxicity (C) for HBF, MDA-MB-231, and ECs, cultured in contact with the dECM and assessed using the LDH assay. **(B)** Bar plot depicting the cell viability (V) of each cell type embedded in the dECM bioink after 5 days in culture. **(C)** Representative maximum intensity projection (MIP) images of HBF, MDA-MB-231, and ECs (labeled in pink, green, and cyan, respectively) in the 3D dECM bioink at days 1 and 7 in culture.

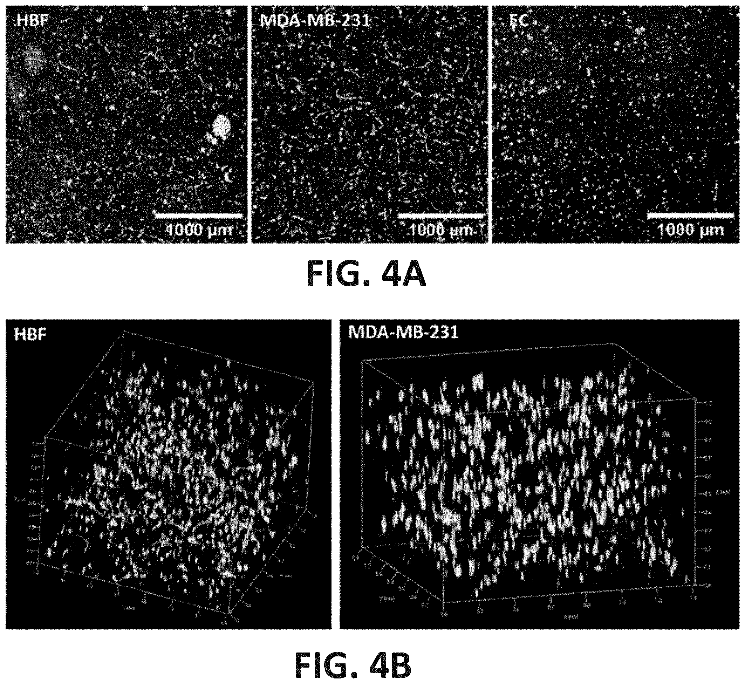

**FIG. 4****: Evaluation of cell viability in the dECM(A)** Representative maximum intensity projection (MIP) live confocal fluorescence microscopy images of HBF, MDA-MB-231 and TeloHAEC cells stained with Calcein (green, viable cells) and Propidium iodide (red, dead cells) after 5 days embedded in dECM 3D culture. **(B)** 3D reconstruction of HBF and MDA-MB-231 cells embedded in dECM 3D culture stained with Calcein (green, viable cells) and Propidium iodide (red, dead cells). Z = 1mm.

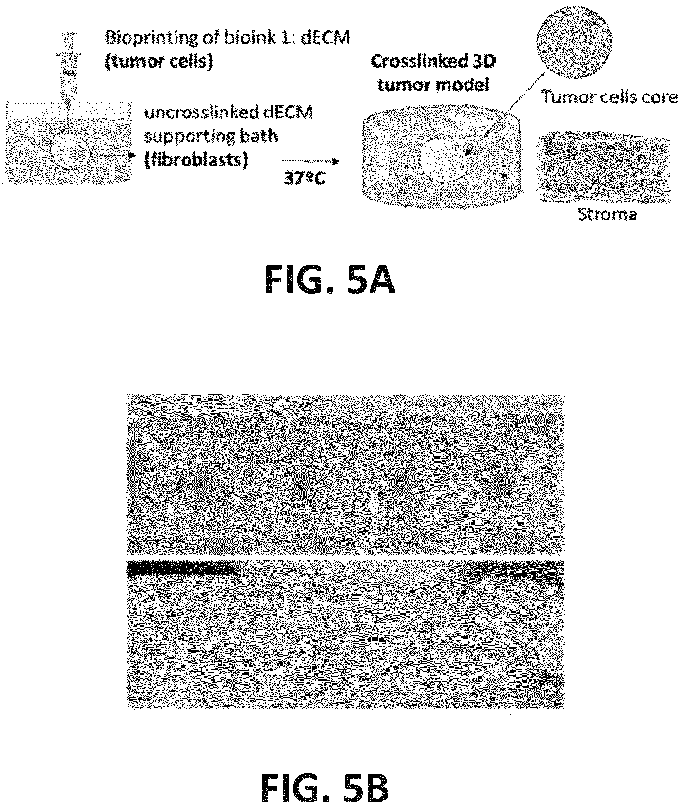

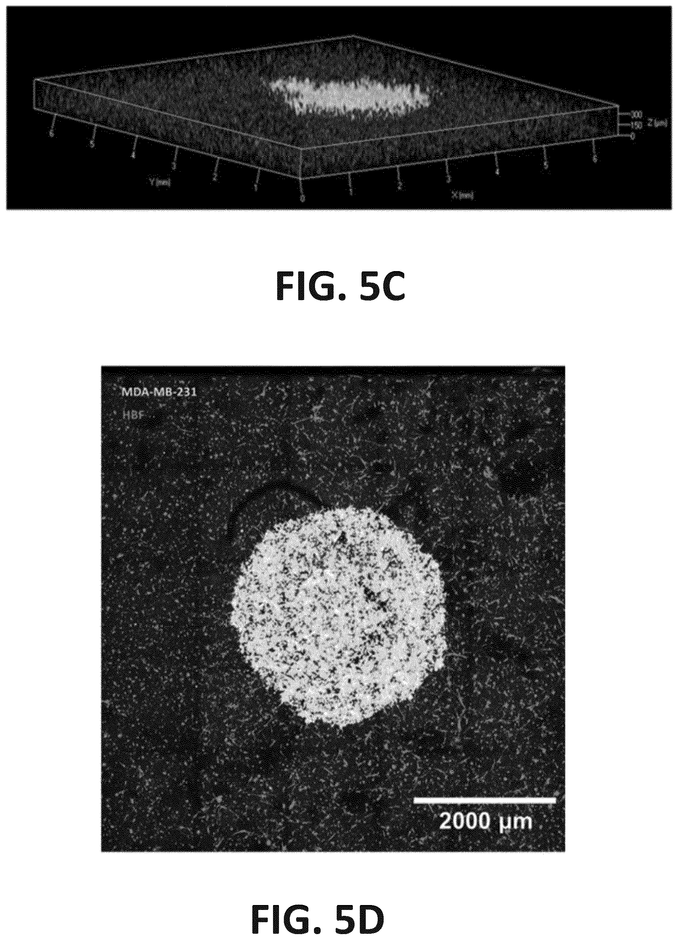

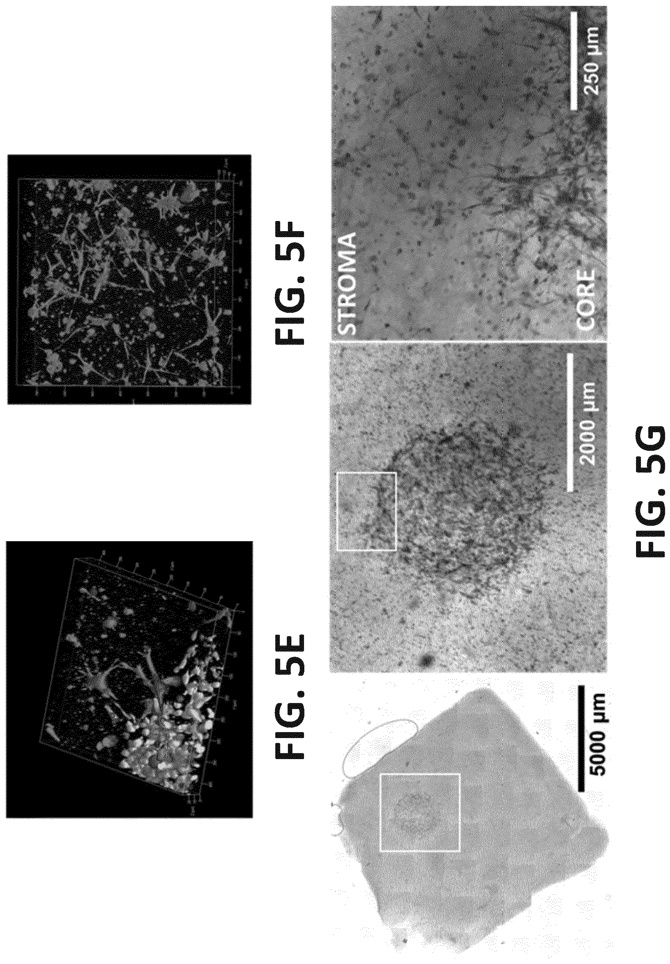

**FIG. 5****: Development of bioprinted tumor-stroma models. (A)** Schematic diagram illustrating the preparation of the bioprinted tumor-stroma construct. **(B)** Representative example of crosslinked tumor-stroma models showing upper and lateral view of the tumor core coloured in blue embedded in the dECM. **(C)** Confocal 3D image reconstruction of a bioprinted 3D tumor-stroma model depicting an inner core composed of MDA-MB-231 breast tumor cells (green) surrounded by HBF (pink). **(D)** Live-cell confocal MIP fluorescence image of 3D tumor-stroma model. **(E)** Confocal 3D image reconstruction depicting interactions between tumor cells and HBF from the stromal compartment **(F)** Confocal 3D image reconstruction showing HBF growing in the stromal compartment of the bioprinted tumor model. **(G)** Representative H&E images of 3D printed tumor-stroma tissue sections after 7 days in culture. Images show both the stromal and tumor core compartments.

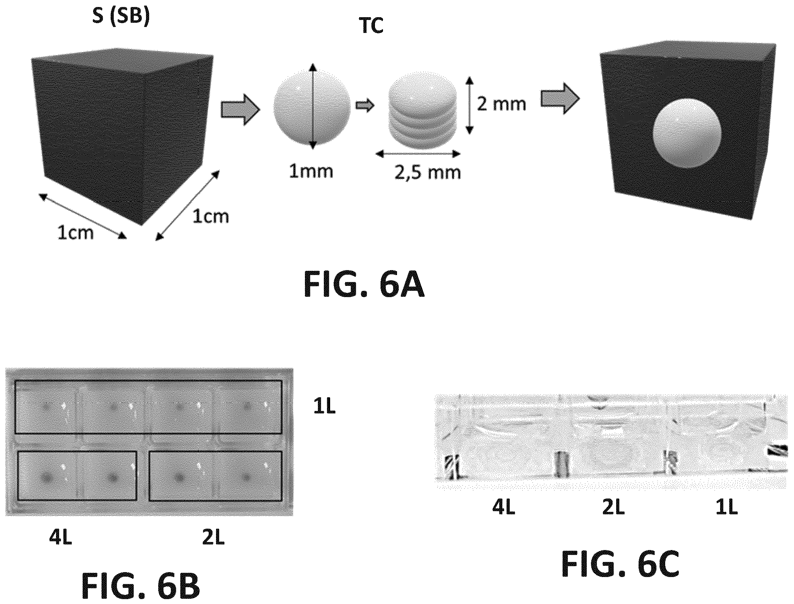

**FIG. 6**: (A) Bioprinting design and architecture of the tumor-stroma models. (S for Stroma, SB for supporting bath, TC for tumor core) **(B)** Top view and **(C)** lateral view of representative photographs depicting the tumor-stroma models and the number of bioprinted cancer cells bioink layers (L). Methylene blue was added to the core bioink to facilitate visualization of the tumor-stroma architecture.

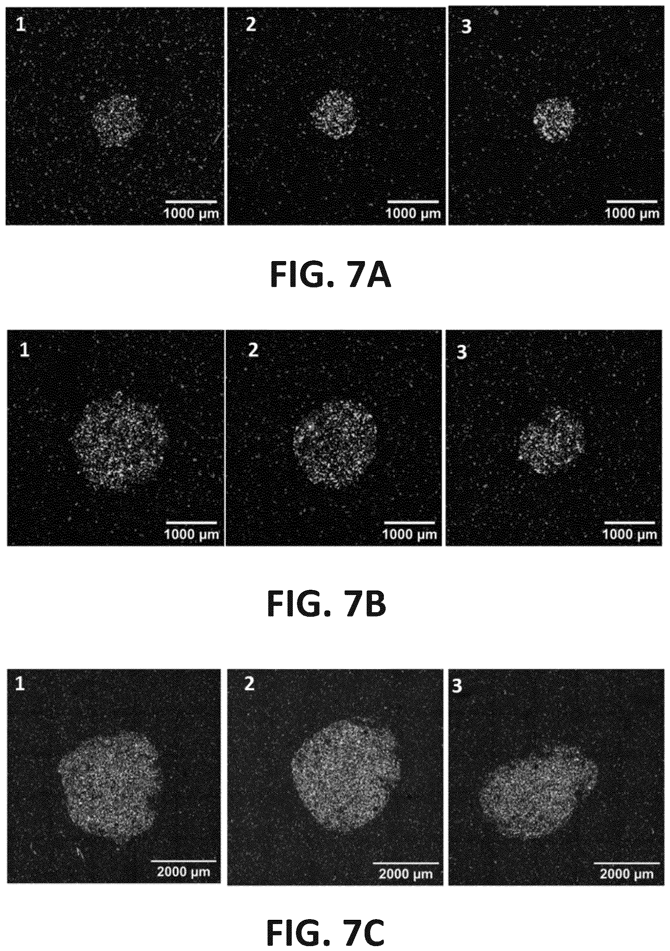

**FIG. 7****:** Representative live confocal MIP image of 3 replicates of bioprinted MDA-MB-231 3D tumor-stroma models exhibiting different tumor core sizes according to the number of cancer cell bioink layers bioprinted **(A)** 1 layer **(B)** 2 layers **(C)** 4 layers.

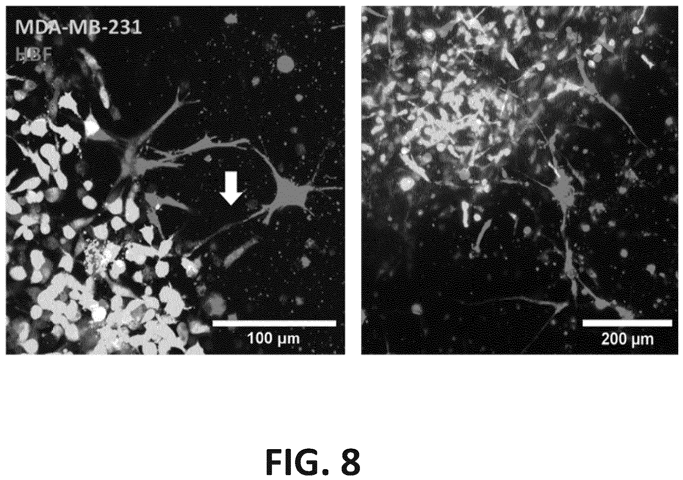

**FIG. 8****:** Representative confocal MIP images depicting interactions between tumor cells (green) of the bioprinted tumor core and HBF (pink) from the stromal compartment. White arrow indicates physical interaction between both cell types.

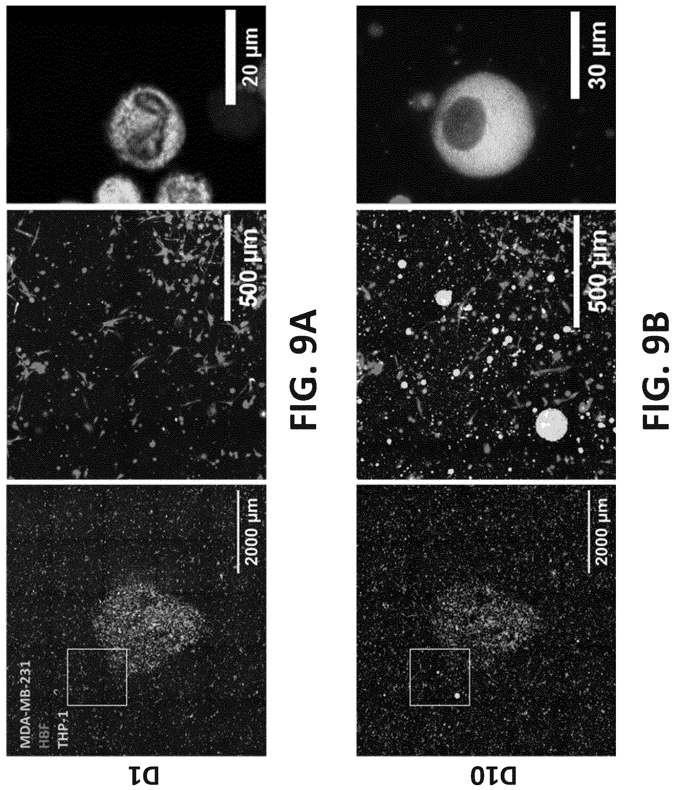

**FIG. 9****: Characterization of THP-1 monocytes evolution within the tumor-stroma model.** Live-cell confocal MIP fluorescence image of 3D TNBC tumor-stroma model incorporating MDA-MB-231 TNBC cells (green), HBF (pink) and THP-1 monocytes (cyan) in the stromal compartment at day 1 **(A)** and at day 10 **(B)** in culture. Insets show magnifications to appreciate the morphology and phenotype evolution of THP-1 monocytes over time.

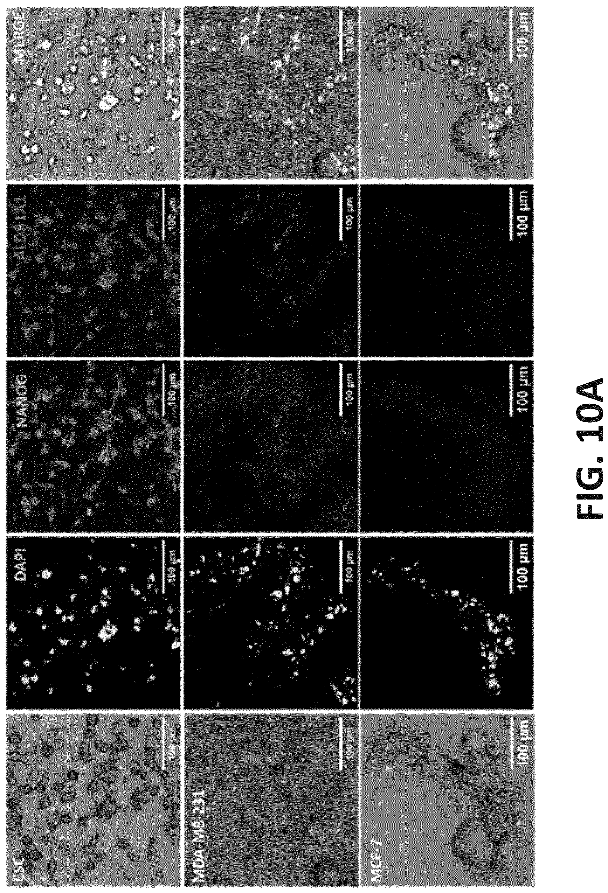

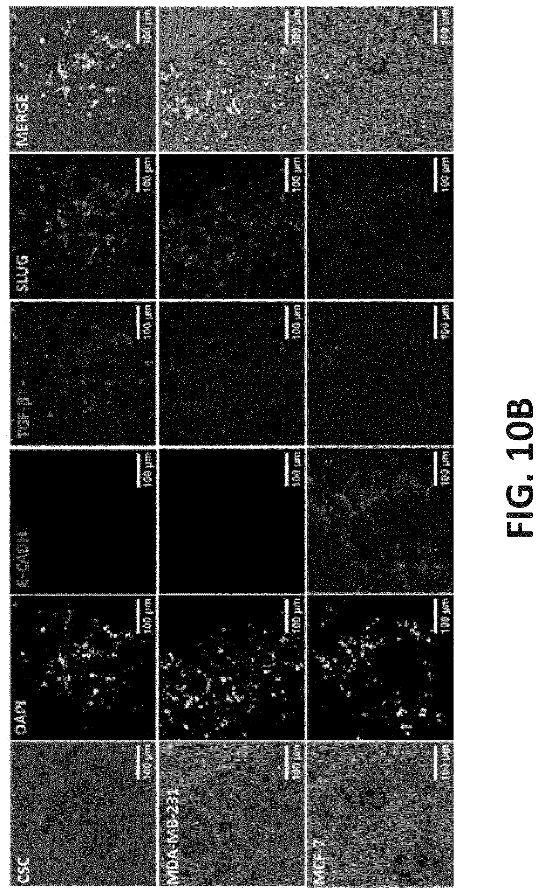

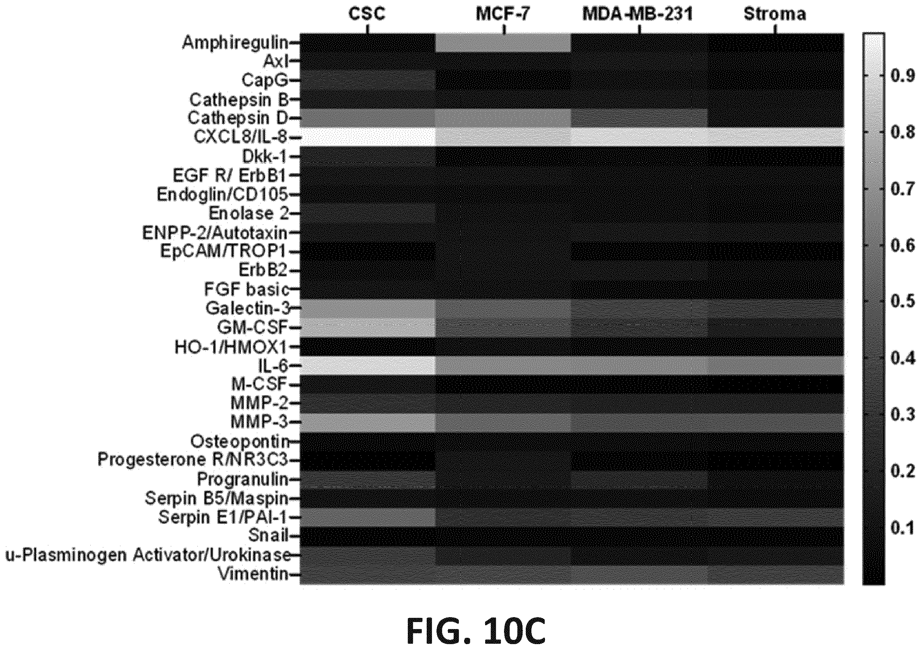

**FIG. 10****: Biological characterization of developed breast tumor-stroma model subtypes. (A)** Representative IF images of the 3D tumor-models stained for NANOG (green) and ALDH1A1 (red), for the analysis of CSC markers in the models and with DAPI (cyan) for cell nuclei. **(B)** Representative IF images of the 3D tumor-stroma models stained for E-CADHERINE (red), TGF-B (pink) and SLUG (green) for the analysis of EMT progression in tumor cells, and with DAPI (cyan) for cell nuclei. **(C)** Heat-map indicating the oncoproteome in tumor-stroma and stroma 3D models, measured using a microarray proteome profiler.

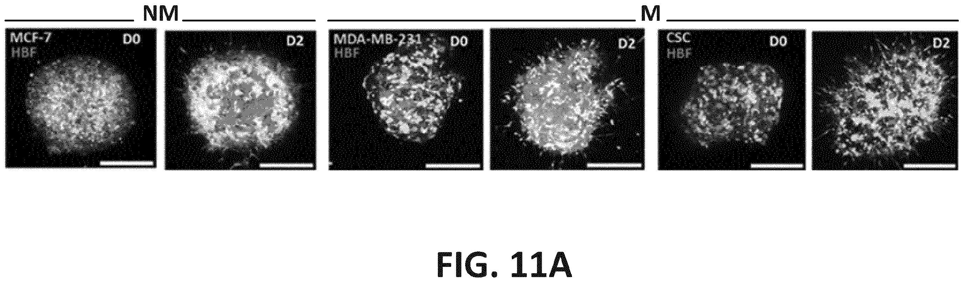

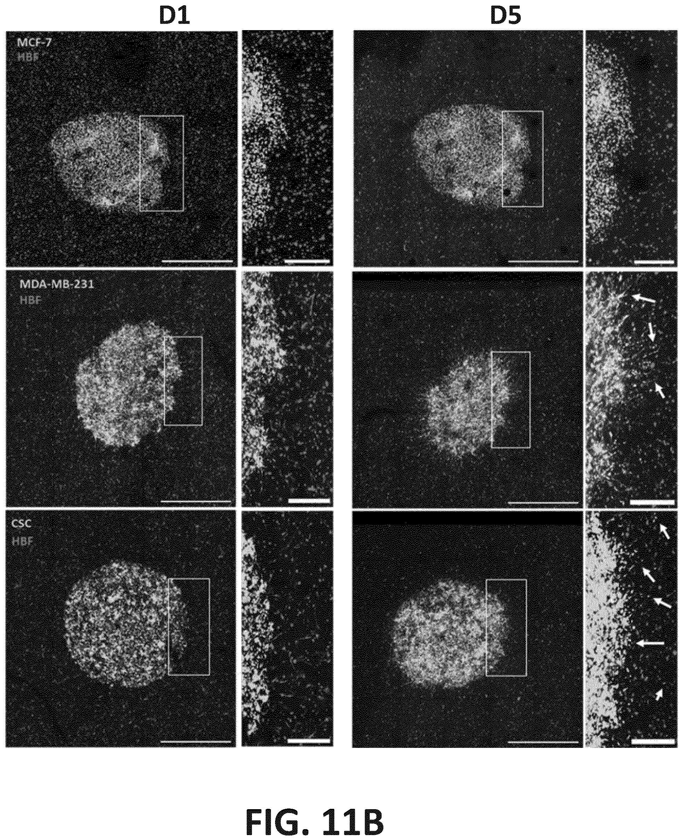

**FIG. 11****: Metastatic potential evaluation of 3D bioprinted tumor-stroma models. (A)** MIP confocal images of MCF-7, MDA-MB-231 and CSC spheroids embedded in dECM at day 0 and 2 in culture. **(B)** MIP confocal images of MCF-7, MDA-MB-231 and CSC tumor-stroma models at day 1 and 5 in culture. (NM refers to non metastatic and M refers to metastatic) White arrows indicate metastatic cells. Scale bar=2000µm.

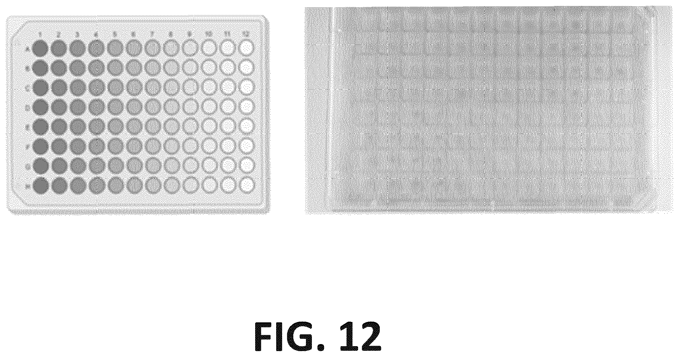

**FIG. 12****:** Example of a high throughput plate containing 96 tumor-stroma models for drug screening assays

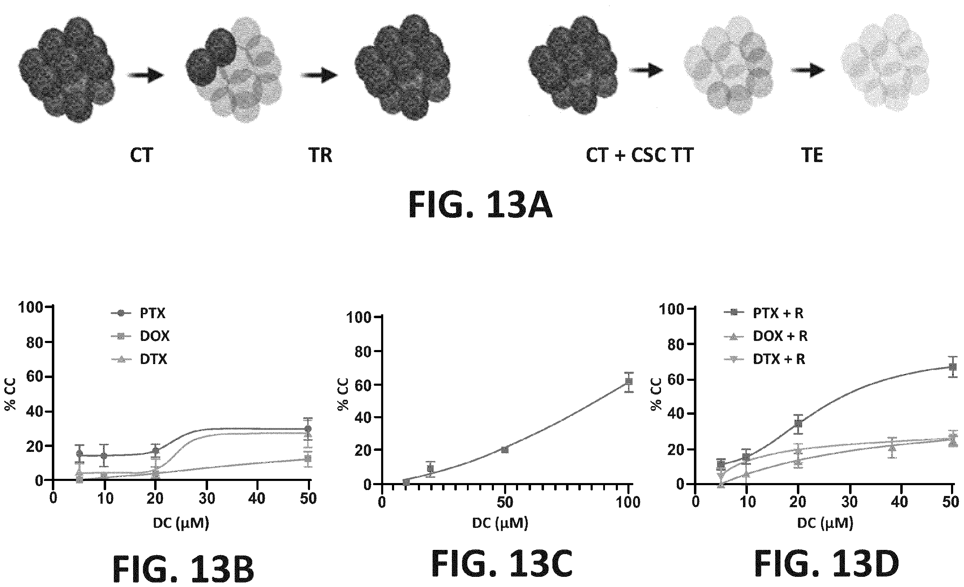

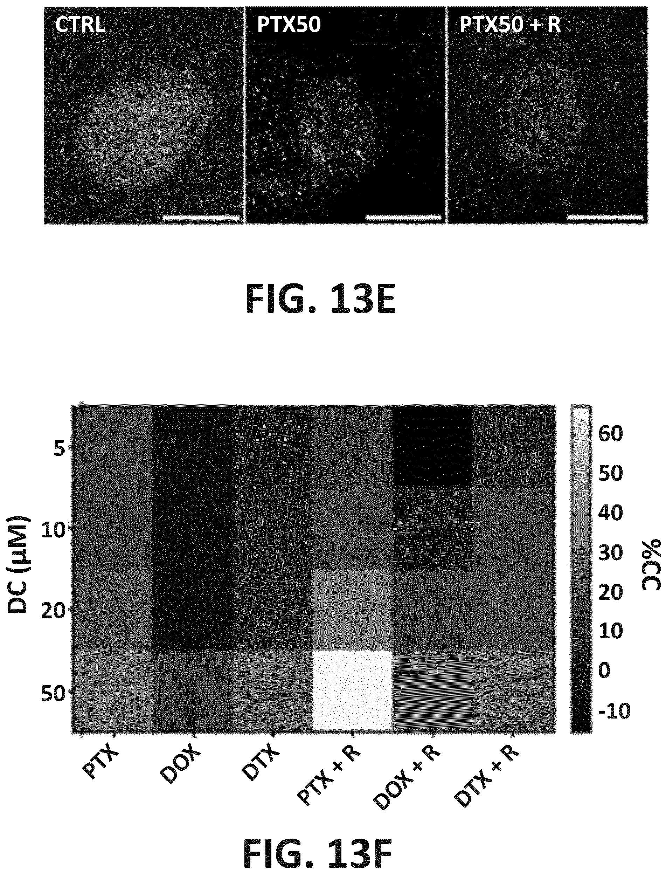

**FIG. 13****: Evaluation of CSC-targeted therapy effectiveness in CSC tumor-stroma models. (A)** Schematic illustration showing therapeutic strategy followed to eliminate CSC populations (CT: Chemotherapy, TR: Tumor Relapse, CSC TT: CSC targeted therapy; TE: tumor erradication). Graphs depicting the drug dose-response curves in terms of cell toxicity (CC, %) in CSC 3D tumor-stroma models, exposed for 72 h to increasing concentrations (DC refers to drug concentration) of Paclitaxel (PTX), Doxorubicin (DOX), and Docetaxel (DTX) **(B),** Reparixin (R) (**C)** and Paclitaxel, Doxorubicin, and Docetaxel and Reparixin (50 µM) (**D). (E)** Representative live confocal fluorescence images of live/dead viability tests on CSC tumor-stroma models exposed to PTX and to PTX+Reparixin (50 µM) for 72 h. (CTRL refers to control)

### OBJECT OF THE INVENTION

The main object of the present invention is a process to prepare a 3D tumor model comprising an inner core completely surrounded in all three dimensions by an outer stromal shell, the process comprising:
a) preparing a supporting bath of a first bioink produced by mixing at least fibroblasts and/or endothelial cells with a dECM in an uncrosslinked state and introducing the mixture in a recipient;
b) preparing a second bioink by mixing at least tumor cells with dECM;
c) introducing an inner core within the supporting bath by bioprinting the second bioink inside the supporting bath prepared in step a) and at a distance of the walls of the recipient to obtain a gel structure formed by the inner core containing the at least tumor cells embedded in dECM completely surrounded in all three dimensions by the outer stromal shell comprising the at least fibroblasts and/or endothelial cells embedded in dECM in an uncrosslinked state; and
d) incubating the gel structure resulting from step c) at a temperature between 25 and 40 °C for the simultaneous crosslinking of the inner core and the outer stromal layer.

The process for preparing the tumor model can be referred to from now on as the process of the invention.

It is also an object of the invention a three-dimensional tumor model obtainable by the process of the invention. This can be referred from now on as "three-dimensional tumor model of the invention", "3D tumor model of the invention", "tumor model of the invention" or simply "the model of the invention".

Further, it is an object of the invention a method for testing anticancer drugs based on the use of a three-dimensional tumor model obtainable by the process of the invention. This can be referred to from now on as the method of the invention.

Finally, it is an object of the invention the use of the bioprinted three-dimensional tumor model obtainable according to the process of the invention to reproduce the cellular heterogeneity, the spatial architecture and the physiopathological complexity of the tumor microenvironment to develop a personalized medicine and for developing new therapies based on the understanding of the tumor progression in a three-dimensional environment mimicking the tumor microenvironment, preferably of metastatic tumors.

### DETAILED DESCRIPTION OF THE INVENTION

The following definitions are provided to assist in understanding and interpreting the present invention:
"Three-dimensional tumor model" or "3D tumor model": it refers to an *in vitro* reproduction in three dimensions of a certain tumor, produced by bioprinting. It represents one of the objects of the invention, and it is formed by two differentiated structures: a) an inner core comprising or consisting essentially of tumor cells within a dECM and b) an outer stromal shell completely surrounding the inner core in all three dimensions comprising or consisting essentially of fibroblasts and/or endothelial cells within a dECM. The dECM acting as matrix for the cells in both the inner core and the stromal shell is preferably free of any exogenous polymer or rheological modifier. Although the basic model is as explained, the inner core and the outer shell may further comprise other types of cells, especially immune cells for reproducing situations that may occur during the progression of a tumor. In the preferred embodiment of the invention, the three-dimensional tumor model of the invention is a 3D breast tumor model.
**"Bioink":** it refers to any formulation used in bioprinting technologies, usually consisting of a biocompatible material and a compulsory cellular component.
**"Exogenous polymer":** it refers to polymers that do not originate inside the dECM. It applies to either biopolymers of animal origin that are added externally to the bioink (collagen, hyaluronic acid, gelatin, Matrigel, etc), modified biopolymers (methacrylated hyaluronic acid or gelatin) or to other polymers of either synthetic (polyethylene glycol derivatives or methacrylates) or natural (cellulose, alginate, etc.) origin.
**"FESH":** it is the acronym of Freeform Embedding of Suspended Hydrogel that refers to a printing strategy that involves the use of a printable bioink or material (supporting bath) to support the printing of a second bioink or material. This strategy allows for the printing of low viscosity materials that would not retain their shape or structure after printing without being embedded in the supporting material.
**"First Bioink":** in the context of the invention, it refers to the bioink giving rise to the outer stromal layer of the 3D tumor model of the invention. Its basic composition comprises the mixture of fibroblasts and/or endothelial cells with dECM, and optionally other cells such as immune cells. In a preferred embodiment, the first bioink comprises fibroblasts, endothelial cells and dECM.
**"Second Bioink":** in the context of the invention, it refers to the bioink used to prepare the inner core of the 3D model of the invention. Its basic composition is a mixture of tumor cells with dECM and optionally other cell types such as immune cells. In a preferred embodiment, the second bioink comprises tumor cells, preferably breast tumor cells.
**"Supporting bath":** in the context of the invention, it refers to the volume of the first bioink in a liquid state contained in a recipient such as a well of a well plate and ready for receiving the second bioink. The fact of the first bioink being in a liquid state allows the bioprinting of the inner core with the second bioink inside the supporting bath. The first bioink filling the small recipient is the one giving rise to the outer stromal shell in the tumor model of the invention.
**"Bioprinted" or "bioprinting:** it refers to the way or technique for producing the 3D tumor model of the invention, which is based on the use of bioinks comprising or consisting essentially of a decellularized matrix (dECM) fraction and a cellular fraction containing the cells. The composition of the cellular fraction of the bioink depends on whether the bioink is intended for the bioprinting of the inner core or of the outer stromal shell. In the context of the invention, any bioprinting machine or apparatus is suitable for producing the 3D tumor model of the invention, as long as it is adapted for bioprinting using the FESH technology.
**"Inner core":** it refers to the internal part of the three-dimensional tumor model obtained by bioprinting the second bioink within the supporting bath following the process of the invention, which comprises or consists essentially of a dECM and tumor cells. The inner core may optionally contain other types of cells such as, for instance, immune cells.
**"Stromal Shell":** it refers to the volume of the first bioink surrounding the inner core of the three-dimensional tumor model obtained by the process of the invention and it comprises or consists essentially of a dECM and fibroblasts and/or endothelial cells. The outer stromal shell may optionally contain other types of cells such as for instance immune cells.
**"Decellullarized extracellular matrix"** or **"dECM":** in the context of the invention, it refers to the substance in which the cells comprised in both the inner core and the stromal shell are embedded and provides structure to the 3D tumor model and support to the cells, as well as allows them to grow three-dimensionally in the 3D tumor model. The dECM is composed mainly of collagens and other types of proteins, as well as of regulators and other bioactive molecules. The dECM bioink is obtained by a precise protocol of decellularization of the tissue of interest and an additional step to solubilize the decellularized tissue and obtain a bioprintable material with liquid properties. The use of dECM derived from the tissue of origin of the tumor ensures the presence of ECM proteins and bioactive molecules found in the normal microenvironment of the tumor.
**"Completely surrounding" or "Completely surrounded":** in the context of the invention, it refers to the disposition of the stromal shell with respect to the inner core. In particular, the expression means that the entire surface of the inner core is covered, coated or surrounded by the outer stromal shell, so that any part of the inner core surface is always in direct contact with the stromal shell. In the context of the invention, this feature is achieved by producing the inner core below the surface of the stromal shell surface and at a distance of the walls of the vessel or recipient that contains the tumor model, this distance being any distance that allows the stromal shell to completely surround the inner core.
**"Uncrosslinked state":** In the context of the invention, it refers to the state of the dECM bioinks before being subjected to the crosslinking of step d) in the process of the invention.
**"Tumor cells":** in the context of the invention, it refers to a type of cancer cells present in the inner core of the 3D tumor model. They can generally be understood as cancer cells that divide continually forming tumors. Any type of tumor cells may be used in the context of the invention but preferably the tumor cells are derived from a patient's tumor or selected from different subtypes of breast tumors, preferably MDA-MB-231, MCF-7 and HCC1806 cell lines, pancreatic tumors, preferably MIA Paca2 and Capan-2 cell lines, lung tumors, preferably H69PR and A549 cell lines, kidney tumors, preferably A-498 and 786-0 cell lines, hepatic tumors, preferably HepG2 and HepT1 cell lines, prostate tumors preferably DU145, PC3, and LNCaP cell lines, ovarian tumors, preferably SK-OV-3, A2780 and OVCAR-3 cell lines.
**"Fibroblasts":** in the context of the invention, it refers to a type of cells present in the outer stromal shell of the 3D tumor model. It is the type of cells that synthesize the extracellular matrix and collagen, produce the structural framework (stroma) for animal tissues, and play a critical role in wound healing. Fibroblasts are the most common cells of connective tissue in animals. Any fibroblast may be used in the context of the invention but preferably fibroblasts are derived from the same source as the tumor cell, i.e. from the own patient. It is also preferred that fibroblasts are derived from the same tissue of origin of the tumor.
**"Endothelial cells":** in the context of the invention, it refers to a type of cells present in the outer stromal shell of the 3D tumor model. It is the type of flat cell that forms the inside of all blood vessels (including capillaries) and is in permanent contact with blood. They act as regulators of cellular and molecular traffic from blood into tissues. Any endothelial cell may be used in the context of the invention but preferably endothelial cells are derived from the same source as the tumor cells, i.e. from the own patient, but also commonly known and established endothelial and fibroblast cell lines may be used, for instance, endothelial HUVEC or HAEC cell lines.
**"Immune cells":** in the context of the invention, it refers to different types of cells that may be present in some embodiments, either in the inner core or in the stromal shell, or in both, within the 3D tumor model. It generally refers to any type of cells from the innate or adaptative immune system that help in the protection of organisms from infections and diseases, including cancer. Any type of immune cells may be used in the model of the invention without limitation but in a preferred embodiment immune cells comprise macrophages, natural killer cells, dendritic cells and T lymphocytes.
**"Tumor derived factors":** generally refers to substances produced by cancer cells that can influence the behavior of surrounding normal cells, blood vessels, and immune cells. These can include cytokines, growth factors, enzymes, and other molecules that affect the tumor microenvironment and contribute to tumor growth, progression, and metastasis. These factors can also influence the behavior of normal cells in the area, such as blood vessels, immune cells, and/or fibroblasts, and modulate the host response to the tumor.
**"Tumor microenvironment or "TME":** it generally refers to all the components and conditions surrounding a tumor *in vivo*, from physical, physiological, biochemical, cellular, and mechanical points of view. The 3D tumor model obtainable by the process of the invention mimics the tumor microenvironment.
**"Regular morphology":** it refers to those embodiments where the inner core of the 3D model of the invention has essentially a geometric shape, preferably a sphere or spheroid. The term spheroid refers to the regular morphology when the geometric shape most closely related to the regular morphology is a sphere.
**"Maximum diameter":** it refers to the maximum distance from one end-point of a sphere or spheroid to an opposite end-point. In the context of the invention Maximum diameter generally refers to the maximum distance from one end-point of the inner core to an opposite end-point of the inner core.
**"Tissue of origin"** or **"tissue of origin of the tumor":** it refers to the biological tissue where the tumor grows in the patient. This expression is applicable to those embodiments where the dECM is obtained from the same tissue where the tumor object of the particular model developed was originated. *Mutatis mutandis* it can also refer to the tissue of origin of the fibroblasts, endothelial cells and/or immune cells.
**"Tumor models of different nature":** in the context of the present invention, it may refer to tumor model developed from different patients, or to tumor models of different type of tumors, such as for instance, breast tumor and pancreatic tumor or even to tumor models from different subtypes of the same type of tumor such as, for instance, HER2-enriched, triple negative, luminal a or luminal b breast cancer.
**"High-throughput testing method", "high-throughput testing" or "high-throughput screening (HTS)":** in the context of the invention, it refers to a method used in drug screening or drug discovery to rapidly test the biological activity of large numbers of compounds on biological samples using automated equipment and techniques. The goal of HTS is to identify potential drug candidates from a large library of compounds and prioritize them for further testing and development. This approach allows for efficient screening of many compounds in a short period of time, leading to faster and more cost-effective drug discovery.

### Process for preparing a bioprinted three-dimensional tumor model

A first aspect of the present invention is a process to prepare a tumor model having an inner core completely surrounded by an outer stromal shell, the process comprising:
a) preparing a supporting bath of a first bioink produced by mixing at least fibroblasts and/or endothelial cells with a dECM in an uncrosslinked state and introducing the mixture in a recipient;
b) preparing a second bioink by mixing at least tumor cells with dECM;
c) introducing an inner core within the supporting bath by bioprinting the second bioink inside the supporting bath prepared in step a) and at a distance of the walls of the recipient to obtain a gel structure formed by the inner core containing the at least tumor cells embedded in dECM completely surrounded in all three dimensions by the outer stromal shell comprising the at least fibroblasts and/or endothelial cells embedded in dECM in an uncrosslinked state; and
d) incubating the gel structure resulting from step c) at a temperature between 25 and 40 °C for the simultaneous crosslinking of the inner core and the outer stromal layer.

This general process described herein can be referred to along the description as the process of the invention.

The process of the invention comprises two steps that are mainly directed to prepare the bioinks used in the preparation of the tumor model (steps a and b), a step directed to the bioprinting itself (step b) and a step directed to fixing the structure of the tumor model in its final shape by crosslinking (step d). The process for preparing the model of the invention is represented in the scheme of figure 5A and 6A.

Two separate bioinks must be prepared to produce the inner core and the outer stromal shell. Bioinks are made by mixing dECM together with the specific cell types, depending on the part of the tumor model that is going to be produced with such bioink.

The dECM and the nature thereof is a very relevant element of the invention. It has various functions. The first one is to serve as support and as a niche for the 3D growth of the cells. On the other hand, it allows reproducing the functional, mechanical, physiological, and biochemical microenvironment of the tumor. The more relevant aspect leading to all advantages of the invention is that the dECM is preferably not supplemented with or is devoid of any kind of exogenous polymer for increasing crosslinkage or rheological modifier that may negatively impact in the recreation of the TME. In addition, the presence of excessive crosslinking agent may interfere in the free movement of cells within the 3D model. This is not desirable in general and specially not in dynamic models where the movement of cells is under analysis, for instance in metastatic tumor models.

The dECM may derive from any particular tissue but is preferably derived from the tissue of origin of the particular tumor type to model because it ensures the presence of ECM proteins and bioactive molecules found in the normal microenvironment of the tumor. The general process for preparing a dECM from a certain tissue is explained below. The particular procedure to prepare a dECM from the tissue of origin when this tissue is breast tissue, is explained in detail in the examples. In a particular embodiment the dECM has a porcine origin. In a further particular and preferred embodiment the dECM is derived from a healthy tissue of origin and is absent of tumor-derived factors.

In step a) of the process, the first bioink for producing the outer stromal layer is prepared and put in a supporting bath in a liquid or semiliquid state (uncrossslinked). The preparation of the bioink comprises mixing the fibroblasts and/or endothelial cells and dECM. It is very relevant to maintain the supporting bath with the first bioink in an uncrosslinked state so that the FESH bioprinting of the inner core (step c of the process of the invention) may be carried out inside the uncrosslinked bioink of the supporting bath. The referred first bioink present in the supporting bath later on becomes the outer stromal layer at the end of the process of the invention.

From an anatomical point of view, stroma is formed by the cells and structures giving support to organs, glands and tissues. Although small differences exist in the stroma depending on their exact origin, all stromas have in common the presence of fibroblast forming the connective tissue and endothelial cells forming the vessels that perfuse the tissues and organs. With the intention of replicating the natural conditions surrounding tumors, the stromal shell in the model of the invention comprises or consists essentially of fibroblasts and/or endothelial cells embedded in a dECM. The presence of fibroblasts and/or endothelial cells (which are the major type of cells in stromal tissues) together with the dECM reproduce the natural microenviroment of the tumor in the model of the invention.

Ideally, fibroblasts and endothelial cells isolated from the patient having the tumor to be tested are present in a preferred embodiment of the invention, however, also commonly known and established endothelial and fibroblasts cell lines may be used. Endothelial cell lines emulating tumoral vascularization will be maintained in the recreation of any stroma subtypes. For instance, endothelial HUVEC or HAEC cell lines can be used in the model of the invention.

Regarding the fibroblastic population, the election of the stromal host tissue cells can vary depending on the tumor subtype recreation. For example, in the case of the recreation of a lung tumor, human lung fibroblasts and lung alveolar epithelial cells (HPAEpiC) can be used. In the case of recreation of a pancreatic tumor, stromal cells will consist of human pancreatic fibroblasts and pancreatic stellate cells (HPaSteC). In the case of a hepatic tumor, hepatic fibroblasts and hepatic stellate cells (HSCs) can be used. In the case of a prostate tumor, resident prostate smooth muscle cells and prostate derived fibroblasts will be used.

In some embodiments additional cells such as immune cells can be introduced in the outer stromal shell. In such cases, the process may include the addition of further cells in the preparation of the first bioink of step a) of the process of the invention, in addition to fibroblasts and endothelial cells. In a particular embodiment of the invention, step a) additionally comprises mixing human immune cells such as macrophages, natural killer cells, dendritic cells, and/or T-lymphocytes, together with fibroblasts and/or endothelial cells and the dECM. Also, the first bioink may optionally comprise other tissue specific stromal cell types including pericytes, adipocytes and/or mesenchymal cells.

The preparation of the second bioink is carried out in step b) of the process of the invention. The second bioink is aimed for bioprinting the inner core of the tumor model. It comprises mixing at least tumor cells with dECM.

In a particular and preferred embodiment of the process of the invention, the tumor cells used in step b) are cells derived from a patient's tumor. Further particular embodiments are those where cells are selected from different subtypes of breast tumors, preferably MDA-MB-231, MCF-7 and HCC1806 cell lines; pancreatic tumors, preferably MIA Paca2 and Capan-2 cell lines; lung tumors, preferably H69PR and A549 cell lines; kidney tumors, preferably A-498 and 786-0 cell lines; hepatic tumors, preferably HepG2 and HepT1 cell lines; prostate tumors, preferably DU145, PC3, and LNCaP cell lines; and ovarian tumors, preferably SK-OV-3, A2780 and OVCAR-3 cell lines. Actually, the preferred embodiment of the invention comprises the use, in step b) of the process, of breast tumor cells derived from a patient's tumor.

For those embodiments where the inner core comprises additional cells such as immune cells, the process may include the addition of further cells in the preparation of the second bioink in step b) in addition to tumor cells. In a particular embodiment of the invention, step b) additionally comprises mixing human immune cells such as macrophages, natural killer cells, dendritic cells, and/or T-lymphocytes, together with the tumor cells and the dECM.

Next step of the process (step c) comprises the bioprinting of the inner core with the second bioink prepared in step b) inside the supporting bath prepared in step a). As explained before, in order to carry out the FESH bioprinting technique, the supporting bath must be in a liquid or semiliquid state. For the bioprinting of the core in one of the embodiments of the invention, a dispensing needle is introduced within the supporting bath and the second bioink is then extruded. Tumor cores can be printed by one single extrusion step or preferably as several layered circular structures in succesive extrusion steps, such as in example 4 where a 4-layered circular structure is printed to form a spheroid inner core (see also figure 6A). The resulting uncrosslinked structure is formed by the inner core containing the at least tumor cells embedded in dECM which is completely surrounded by the outer stromal shell comprising the at least fibroblasts and/or endothelial cells embedded in dECM.

After the bioprinting step, the uncrosslinked structure resulting from step c) is then subjected to an incubation at a temperature between 25 and 40 °C for the simultaneous crosslinking of the inner core and the outer stromal layer. After this crosslinking step (step d) the 3D tumor model of the invention is obtained. The crosslinking step gives the model mechanical properties similar to those observed in real tumors.

The bioprinting of the inner core may be made with any desired morphology, be it regular or irregular. In a particular and preferred embodiment, it may be printed with a regular shape, such as a sphere or spheroid. This embodiment may represent the first stages of tumorigenesis and may allow to monitor tumor progression over time.

In a more particular embodiment, the bioprinting of the inner core can be carried out with an irregular shape, as can be the situation of a more advanced tumor stage.

Reproducing the exact shape of the tumor of a patient in terms of morphology and volume allows for a more realistic assessment of patient tumor evolution and progression and has the advantage of helping to design the better therapeutic approach for a specific patient's tumor.

Although, any tumor size can be reproduced, in a preferred embodiment the maximum diameter of the inner core is between 1 mm and 6 mm of maximum diameter, preferably 1 to 4 mm and more, more preferably from 1 mm to 3.5 mm of maximum diameter, still more preferably 1 to 3 mm of maximum diameter. The complete three-dimensional tumor model including inner core and outer shell can have any volume, but a preferred embodiment the model has a volume in the range 10 mm³ to 2 cm³, preferably in the range of 6 mm³ to 1 cm³.

In the case where the particular dECM used in the preparation of the first and second bioinks discussed above is not purchased from a commercial source, the dECM can be prepared prior to the steps a) and b) of bioinks production. In such as case, it is preferred and recommended to prepare the dECM from the same tissue of origin as the tumor to be modeled. For instance, if a breast tumor model is to be prepared, it is desirable to use breast tissue to prepare the dECM.

The production of dECM bioink from a certain tissue comprises:
i. Dissecting and slicing the tissue of origin of the tumor; e.g., breast tissue;
ii. Washing the tissue resulting from step i) with saline buffer;
iii. Freezing the resulting tissue from step ii);
iv. Treating the resulting tissue from step iii) with 0.5-1 % anionic surfactants; and/or bile acids, preferably for 48-92 hours at 18-25 °C;
v. Washing the resulting tissue from step iv) with water, preferably for 1-2 hours at 18-25 °C;
vi. Treating the resulting tissue from step v) with nucleases, preferably for 12-24 hours at 30-40 °C;
vii. Washing the resulting tissue from step vi) with water, preferably for 1-2 hours at 18-25 °C;
viii. Treating the resulting tissue from step vii) with organic solvents, preferably for 6-24 hours at 18-25 °C;
ix. Washing the resulting tissue from step viii) with saline buffer, preferably at 18-37 °C;
x. Sterilizing the resulting tissue from step ix);
xi. Freeze-drying and milling the resulting tissue from step x);
xii. Performing an acidic digestion of the powder resulting from step xi); and
xiii. Neutralizing the pH of the product resulting from step xii).

In a particular embodiment of the invention, the dECM produced is from porcine origin. In another particular embodiment, the dECM is derived from breast tissue and more particularly from porcine breast tissue as disclosed in examples 1 and 3 below.

### Bioprinted three-dimensional tumor model

In its second aspect, the invention refers to a tumor model obtainable by a process according to the invention.

The three-dimensional tumor model so obtained comprises:
a) an inner core comprising at least tumor cells embedded in dECM; and
b) an outer stromal shell comprising at least fibroblasts and/or endothelial cells embedded in dECM completely surrounding the inner core;

This bioprinted three-dimensional tumor model can be referred to indistinctly along the present specification as "bioprinted tumor model of the invention", "bioprinted model of the invention", the "three-dimensional tumor model of the invention", "three-dimensional model of the invention", "tumor model of the invention" or simply "model of the invention".

Importantly, in a preferred embodiment of the invention the three-dimensional tumor model of the invention is obtained from non-modified dECM-based bioinks. The absence of rheological modifiers and extra crosslinking mechanisms (UV, NaCl), potentially interfering cell behaviour, allows an accurate reproduction of the TME. The model of the invention not only preserves multiple tumor, stromal and immune cell's physiological phenotypic properties, it also allows the free movement of cells along the entire bioprinted model. In particular, the absence of exogenous crosslinking biopolymers that increase the matrix crosslinking in the bioinks facilitates the movement between the inner core and the stromal shell. This makes the model of the invention especially suited for reproduction and analysis of the behaviour of metastatic and non-metastatic tumor cells. Actually, in a particular embodiment, the tumor model of the invention is a mestastatic tumor model

The model of the invention is a 3D structure with two clearly differentiated parts, namely an inner core and an outer stromal shell.

The first one is the inner core and comprises or consists essentially of tumor cells embedded in a dECM. The type of tumor model will mainly depend on the type of tumor cells used for the preparation of the model of the invention. For instance, a breast tumor model can be developed according to the invention by using breast tumor cells in the preparation of the inner core.

In the preferred embodiment of the invention, the tumor cells are derived from a patient's tumor such that the model allows to assess and develop a specific and personalized therapy against the particular patient's tumor.

Although any type of tumor model may be prepared, in a particular embodiment of the invention, the tumor cells are either cells derived from a patient's tumor or commercial cells selected from different subtypes of breast tumors, preferably MDA-MB-231, MCF-7 and HCC1806 cell lines; pancreatic tumors, preferably MIA Paca2 and Capan-2 cell lines; lung tumors, preferably H69PR and A549 cell lines; kidney tumors, preferably A-498 and 786-O cell lines; hepatic tumors, preferably HepG2 and HepT1 cell lines; prostate tumors, preferably DU145, PC3, and LNCaP cell lines; and ovarian tumors, preferably SK-OV-3, A2780 and OVCAR-3 cell lines.

In a preferred embodiment of the invention, the tumor model of the invention is a breast tumor model based on the use of breast tumor cells, preferably derived from a patient suffering from breast cancer.

The second structurally differentiated part of the 3D model of the invention is the outer stromal shell, which comprises or consists essentially of fibroblasts and/or endothelial cells embedded in a dECM which completely surrounds the inner core.

Although small differences exist in the stroma depending on their exact origin, all stromas have in common the presence of fibroblast forming the connective tissue and endothelial cells forming the vessels that perfuse the tissues and organs. In this sense, the stromal shell in the model of the invention comprises or consists essentially of fibroblasts and/or endothelial cells embedded in a dECM. The presence of fibroblasts and/or endothelial cells together with the dECM reproduce the natural microenviroment of the tumor in the model of the invention.

Ideally, fibroblasts and endothelial cells isolated from the patient having the tumor to be tested are present in a preferred embodiment of the invention, however, also commonly known and established endothelial and fibroblasts cell lines may be used. Endothelial cell lines emulating tumoral vascularization will be maintained in the recreation of any stroma subtypes. For instance, endothelial HUVEC or HAEC cell lines can be used in the model of the invention.

Regarding the fibroblastic population, the election of the stromal host tissue cells can vary depending on the tumor subtype recreation. For example, in the case of the recreation of a lung tumor, human lung fibroblasts and lung alveolar epithelial cells (HPAEpiC) can be used. In the case of recreation of a pancreatic tumor, stromal cells will consist of human pancreatic fibroblasts and pancreatic stellate cells (HPaSteC). In the case of a hepatic tumor, hepatic fibroblasts and hepatic stellate cells (HSCs) can be used. In the case of a prostate tumor, resident prostate smooth muscle cells and prostate derived fibroblasts will be used.

The dECM and the nature thereof is also a very relevant element of the invention. It has various functions. The first one is to serve as support and as a niche for the 3D growth of the cells. On the other hand, it allows reproducing the functional, mechanical, physiological, and biochemical microenvironment of the tumor. As explained above, in a preferred embodiment, the dECM is derived from the tissue of origin of the specific tumor model designed. For instance, if a breast tumor model is to be prepared, it is preferred that the used dECM is derived from breast tissue. The fact of using dECM derived from the tissue of origin of the tumor ensures the presence of ECM proteins and bioactive molecules found in the normal microenvironment of the tumor (see for instance Figure 2E).

In addition, the dECMs may be prepared from tissues of many mammalian species, however, in a preferred embodiment the tissue used for preparing the dECM is porcine tissue. On the one hand, porcine tissue is very similar to human from a physiological and biochemical point of view, and, on the other hand, it ensures that the dECM is absent from tumor derived factors, which could be present if the patient tissue is used to prepare the dECM. It is desirable that the dECM is absent from tumor derived factors because they can activate specific oncogenic signaling that dictates genotypic and phenotypic changes in recipient cells embedded in the dECM that do not correspond to intrinsic nature of the tumor cells under study, thus hijacking the potential aggressiveness of tumor cells. In the case of a breast tumor model, the dECM can also be prepared from wastes of human breast tissue resulting from surgery, such as breast reduction or other.

In a preferred embodiment, the dECM used in preparing the tumor model is completely free of exogenous additives such as polymers to enhance the crosslinking of the dECM and modify its mechanical properties. As explained before, the use of the method of the invention where the FESH technique is applied in the bioprinting of the inner core in a liquid or semiliquid supporting bath makes it possible to dispense from using this type of exogenous additives or rheological modifiers. The use of the process of the invention in the preparation of the 3D tumor model of the invention provide the model with mechanical properties similar to those observed in tumors *in vivo.*

From the morphological point of view, the bioprinted three-dimensional tumor model of the invention may have a regular or irregular morphology. In a particular and preferred embodiment, the inner core has a regular shape such as a sphere or spheroid. However, natural tumors may have different shapes. The bioprinting technology allows for the production of inner cores of the tumor model having any regular or irregular shape, thus reproducing the specific shape of a patient's tumor.

In terms of dimensions, the skilled person will appreciate that different dimensions can be achieved by varying the volumes of the bioprinted bioinks, however, and for ease of use, in a preferred embodiment, the model of the invention has an inner core with a maximum diameter in the range of 1 to 6 mm, preferably 1 mm to 4 mm, more preferably from 1.5 mm to 3.5 mm, still more preferably 1 to 3 mm. And the complete three-dimensional tumor model has a volume in the range of 10 mm³ to 2 cm³, preferably in the range of 6 mm³ to 1 cm³, more preferably from 0.3 cm³ to 1 cm³.

Apart from the essential elements conforming the basic model of the present invention, both the inner core and the stromal shell may contain additionally immune cells for *in vitro* mimicking naturally occurring situations. Immune cells play an important role in tumor progression and the presence thereof may reproduce different situations of early or advanced stages of a tumor. In early stages of cancer progression, immune cells are capable of mediating anti-tumor immune response. However, tumor cells acquire the ability to hijack the immune system and favor immune escape, creating an immunosuppressive microenvironment that promotes tumor growth.

In a particular embodiment of the invention, the inner core and the stromal shell may contain an immune cell component. Immune cells may comprise macrophages, natural killer cells, dendritic cells, and/or T lymphocytes. The incorporation of immune cell subsets to the model might allow the study of the immunosuppression process associated with tumor progression at different stages.

### Method for testing anticancer drugs

A further aspect of the invention is represented by a method for testing anticancer drugs comprising the step of contacting the drug to be tested with a bioprinted three-dimensional tumor model according to the invention.

This general method can be referred to throughout the present description as the "testing method of the invention" or simply as "the method of the invention".

The testing method of the invention allows assessing the response of a certain tumor model to a certain drug. The method allows the study of a therapy or a pharmacological treatment during a period of time by the direct analysis of the progression or regression of cell growth in a certain tumor model. This method is extremely useful in research, such as for designing new and innovative therapies against different types of tumorigenic diseases and especially in designing a personalized medicine against a particular patient's tumor.

As the bioprinting technology allows for a multiplicity of reproductions of a tumor model with the same characteristics, in a particular embodiment the method according to the invention is a high-throughput test wherein replicas of a three-dimensional tumor model with the same dimensions and morphology are systematically bioprinted to form an array of tumor models and the test is carried out with multiple drugs, each being contacted with a different tumor model replica, at the same time. This embodiment allows for simultaneously testing, in a multi-well plate, a high number of drugs to determine which are the most effective drugs against a certain tumor and/or the effects of these drugs on the tumor progression. This particular embodiment is an important step in the development of personalized therapies against a patient's tumor.

A further embodiment of the testing method of the invention is a high-throughput testing wherein several three-dimensional tumor models differing in one or more parameters such as the donor patient tumor cells or any other parameter, are tested at the same time against the same drug. This embodiment allows for simultaneously testing a single drug towards several tumor models and appears extremely useful in the research and discovery of new drugs and therapies in pharmacology.

### Use of the bioprinted three-dimensional tumor model

A final aspect of the invention, which is somehow linked to the previous one, is the use of the bioprinted three-dimensional tumor model of the invention to reproduce the cellular heterogeneity, the spatial architecture and physiopathological complexity of the tumor microenvironment to develop a personalized medicine and for developing new therapies based on the understanding of the tumor progression in a three-dimensional environment mimicking the tumor microenvironment. The model of the invention is specially suited for understanding tumor progression in metastatic tumors.

In other words, the three-dimensional tumor model of the invention is a powerful research tool that not only allows for real-time analysis of what happens in the progression of a certain tumor as a physio-pathologic event, but also for the development of new therapies and for adapting and improving existing ones.

Having the three-dimensional tumor model that mimics the tumor microenvironment has the advantage of reproducing the natural conditions of a certain tumor *in vitro,* which represents a great step forward in cancer research.

It is also a very important step in the development of personalized therapies based on the direct *in vitro* pharmacological testing on representative models of a patient's tumor, not only with regard to the morphology and volume of the tumor but also in the microenvironment of the tumor itself.

### EXAMPLES

The following examples disclose the preparation, characterization and validation of a three-dimensional breast tumor model comprising the features of the invention. This specific tumor model is herein described only as a particular and preferred embodiment, but any similar model based on any other type of tumor may be prepared by a skilled person following the indications of the examples presented herein.

In this respect, the present examples do not intend to limit the scope of the present invention in any way.

### Example 1: Preparation of decellularized porcine breast ECM

### 1.1) Obtaining breast tissue

Porcine breast tissue was obtained from a local slaughterhouse. Tissue samples were washed and frozen at -80 °C until use.

### 1.2) Decellularization of porcine breast tissue

Tissues were cut in 1 cm³ pieces and washed with a phosphate buffer (PBS) prior to a freeze/thawing step.

In order to effectively decellularize the tissue, tissue fractions were subjected to 0.5-1% anionic surfactants for 48-92 hours, under orbital shaking (180 rpm) at room temperature.

Thereafter, to eliminate residual reagents, washing steps were carried out between treatments with PBS or distilled water under orbital shaking (180 rpm) at room temperature.

Removal of residual DNA from tissue fractions was carried out by treatment with nucleases (40U/mL) for 12-24 hours at 37 °C in a 1.5 mM magnesium chloride buffer as an enzyme cofactor.

After rinsing the tissue resulting from the previous step, it was treated with organic solvents for 6-24 hours prior to a final PBS and distilled water washing step and a sterilizing step under orbital shaking (180 rpm) at room temperature.

The final product is obtained by freeze-drying and milling the dECM.

### 1.3) Demonstration of the decellularization

Formalin-fixed native ECM and dECMs were embedded in optimal cutting temperature (OCT) compound and cryo-sectioned in 7 µm sections for histological evaluation. These sections were stained by hematoxylin/eosin following a procedure consisting of alcohol re-hydration to distilled water process prior to 3 minutes hematoxylin treatment. Then, sections were immersed in warm tap water and 15 seconds of eosin treatment. Finally, tissue sections were de-hydrated with alcohol and xylene and mounted in DPX mounting media.

Re-hydrated tissue sections were also subjected to permeabilization by incubating them with 0.3% triton X-100 solution for 30 minutes, stained with 1 µg/mL DAPI for 30 minutes and mounted in Fluoromount-G.

7 µm-thick cryo-sections were hydrated in water and submerged in propylenglycol for 2 minutes for delipidation evaluation. Subsequently, tissue sections were stained with Oil Red O for 16 hours and developed with 85% propylenglycol for 1 minute. Prior to mounting in Mowiol 4-88, 2 washings with distilled water were performed.

Remnant single and double strain DNA was analyzed Quant-iT PicoGreen kit following the manufacturers' instructions and including calf thymus DNA as standard. The DNA was extracted from the digestion with proteinase K of the frozen dECMs and native tissues by ethanol precipitation. Detection was carried out in Appliskan multimode microplate reader (Thermo Scientific).

### 1.4) Results

1. Summary of the dECM ink obtention process is shown in FIG.1 where structure A corresponds to the native tissue, structure B corresponds to decellularized ECM, structure C corresponds to dECM powder, and structure D corresponds to dECM ink.
2. Referring to FIG. 2A HE staining showed the preservation of the ECM structure, while both HE and DAPI demonstrated the absence of nuclei in the dECM as compared to ECM. Oil Red O staining showed significant de-lipidation.
3. All the assayed dECMs showed very low remnant DNA (<50 ng/mg tissue) meeting the decellularization criteria established by Crapo *et al.* (Crapo PM *et al.* 2011), as depicted in FIG. 2B.

### Example 2: Breast dECM ink characterization

### 2.1) Rheology of the dECM

The resulting dECM inks were assessed rheologically on a MCR302 rheometer equipped with either a 25 mm diameter cone and plate geometry, a Peltier plate at 10°C, and a humidity chamber to maintain the samples hydrated during the measurements. The excess material was trimmed before the measurements began.

### 2.2) SEM microscopy characterization

Cell-free hydrogels were cross-linked for 30 minutes at 37 °C. and freeze-dried before coating them with a thin gold/palladium layer by magnetron sputtering before being imaged with a JSM-6490LV microscope (Jeol) with a working distance of 10 mm and 15 kV voltage.

### 2.3) Proteomic characterization

Samples were treated following the protocol described by Wisniewski et al. [Wisniewski 2009] with minor modifications and analyzed in a hybrid trapped ion mobility spectrometry - quadrupole time of flight mass spectrometer (timsTOF Pro with PASEF, Bruker Daltonics) coupled online to a nanoElute liquid chromatograph (Bruker). The sample (200 ng) was directly loaded in a 15 cm Bruker nanoElute FIFTEEN C18 analytical column (Bruker) and resolved at 400 nL/min with a 100 min gradient. The column was heated to 50 °C using an oven. Protein identification and quantification was carried out using MaxQuant software using default settings [Cox 2008]. Searches were carried out against a database consisting of pig protein entries (Uniprot/Swissprot+TrEMBL), with precursor and fragment tolerances of 20 ppm and 0.05 Da. Only proteins identified with at least two peptides at FDR<1% were considered for further analysis.

### 2.4) Biocompatibility of dECM inks

Biocompatibility of the dECM inks was demonstrated by CellTox Green kit, Cell Counting Kit 8 following manufacturers' instructions by culturing Hs27 cells with the previously crosslinked dECMs for 24, 48 and 72 hours.

### 2.5) Results

1. The rheological properties of the ink were assessed in order to define the printing conditions as well as the choice of the printing method. The breast dECM ink presented a thermally induced gelation behavior starting around 25 °C (FIG. 2C left) and showed a viscoelastic behavior (FIG. 2C right) as the storage modulus (G') was greater than the loss modulus (G") within the frequency sweep. Also, the dECM ink had a shear thinning behavior (FIG. 2D) (viscosity decreases as shear rate increases) meaning this material could be finely printed by pneumatic extrusion.

After incubating cell-free gels for 30 minutes at 37 °C, both G' and G" increased and were stable through time, indicating that a stable module is achieved regardless of time and frequency (FIG. 2C right).

2. The proteomic characterization of the native ECM, dECM and ink revealed that the decellularization protocol partially changes the overall protein composition of the matrix, although most of the components are maintained (FIG. 2E and F). Less peptides were identified in the ink product, as the previous digestion step may be interfering the generation of tryptical peptides required for the LC-MS/MS technique, revealing a complex biochemical composition though.

3. We observed that the dECM exerted background levels of cytotoxicity on all three cell populations, after 72 h in culture. Notably, EC displayed reduced levels of cytotoxicity compared to the control. After 7 days in culture, the samples were stained with live/dead fluorophores to determine the proportion of each population, indicating that cell viability was above 80% (Fig. 3 and 4). Additionally, by staining cells with CellTracker fluorophores prior to embedding in the bioink, cell proliferation and distribution could be studied over time. Cells were homogeneously distributed in the gels and demonstrated increased proliferation and expansion over time within the dECM 3D culture, thereby confirming the biocompatible nature of this material (Fig. 3C). For completion, we examined the behavior of cells inside the dECM bioink. Cells embedded in dECM bioink featured cellular filopodia extending towards the surrounding matrix, a clear sign of cell-dECM interactions, which is crucial to correctly mimic the TME

### Example 3: Formulation of the dECM inks

The powder resulting from the milling step in example 1 was digested in 1 mg/mL pepsin in 0.5 M acetic acid to a final concentration of 20 mg/mL for 48 hours at constant stirring. After two days, pH was neutralized with 10M NaOH.

In order to obtain the inner core bioink (second bioink), neutralized dECM ink was mixed with human breast cancer cells (MDA-MB-231 and MCF- 7 cells).

In order to obtain the stromal shell bioink(first bioink), neutralized dECM ink was mixed with human breast fibroblasts (HBF), aortic endothelial cells (TeloHAEC) and THP-1 monocytes.

### Example 4: Bioprinted 3D Human Breast Tumor-Stromal

### 4.1) Cell Culture

Primary human breast fibroblasts (HBF) were purchased from Innoprot (Derio, Spain) and human breast MDA-MB-231, MCF-7 and THP-1 cell lines were obtained from American Type Culture Collection (ATCC, HTB-26 and HTB-22, respectively). MDA-MB-231 ALDH:tdTomato cell lines were generated as previously described (Gener et al., 2015, 2019; González-Callejo et al., 2023). CSC subpopulations from MDA-MB-231 ALDH:tdTomato were isolated according to their tdTomato expression in a FACS Aria cell sorter (BD Biosciences, Franklin Lakes, NJ). These cell lines were cultured in DMEM supplemented with 10% FBS with 1% penicillin/streptomycin (Fisher Scientific). All cell lines cultures were grown in standard tissue culture conditions at 37 °C and 5% CO₂.

### 4.2) Biocompatibility of dECM gels

To assess cell viability within the dECM bioink, cells were mixed with the dECM bioink at a concentration of 5 × 10⁵ cells/mL, seeded in Ibidi 8-well plates, and maintained in culture for 5 days. Then, cell viability was monitored using a live/dead cell assay. 3D cell cultures were incubated with Calcein AM (1/100) and PI (1/50) for 20 min. The corresponding confocal images were analyzed.

### 4.3) Cell fluorescent labelling

Prior to bioprinting, cells were fluorescently labelled with different emission cell trackers. Cancer cells were stained with CellTracker Green CMFDA, HBF with CellTracker Far red and THP-1 cells with CellTracker Blue (ThermoFisher) in order to assess 3D architecture and relative positions of cell types in the construct.

### 4.4) Bioprinting

The tumor compartment of the constructs comprised 100% MCF-7, MDA-MB-231 or MDA-MB-231 isolated CSC cells. The stromal compartment comprised 100% HBF or 50% HBF and 50% THP-1 cells (cell number ratio) depending of the assay performed.

Prior to bioprinting, cells were fluorescently labelled with different fluorescent cell trackers (CellTracker^{™}, ThermoFisher). Cancer cells were stained with CellTracker^{™} Green CMFDA Dye, HBFs with CellTracker^{™} Deep Red, and THP-1 cells with CellTracker^{™} Blue CMF₂HC Dye. For the stromal compartment bioink formulation, either HBF or HBF and THP-1 cells were mixed in the appropriate proportions in the dECM ink at a concentration of 1 × 10⁷ cells/mL. For the tumor compartment, cancer cells were mixed in the dECM bioink at a 2 × 10⁷ cells/mL and bioprinted within the 1 cm² of an Ibidi 8-well chamber slides (Ibidi) previously filled with the first bioink. Tumor cores were printed as 4-layered circular structures with a printing design of 1 mm in diameter (Figure 6A). dECM bioinks were extruded through a dispensing needle with an internal diameter of 0.3 mm (Nordson) at 10 °C, pressures of 0.010-0.012 MPa, and printing speeds of 0.5-1 mm/s. Immediately following bioprinting, tumor-stroma models were placed at 37 °C to promote dECM bioink cross-linking. Resulting tumor-stroma constructs measured 1 cm × 1 cm × 3 mm. After 20 min, complete DMEM was added to the culture plates. Constructs were then incubated for up to 14 days.

### 4.5) Microscopy

All confocal images were taken using a Zeiss LSM 880 confocal laser scanning microscope equipped with 405 nm (blue fluorophore excitation), 488 nm (green fluorophore excitation) and 633 nm (far red fluorophore excitation), and Plan-Apochromat 10× objective (0.45 N.A.) and Plan-Apochromat 20× objective (0.8 N.A.) In the case of 3D characterization of tumor constructs, Z-stacks of approximately 50 µm in thickness were obtained and post-imaging 3-pixel median filter applied prior to 3D rendering, to obtain images from different angles.

### 4.6) Results

1. Cell viability in the dECM is shown in FIG. 3 and 4, showing high cell density and cell viability at day 5 in culture (high % of green cells).
2. Bioprinted breast tumor cell constructs were developed by the bioprinting technology assisted generation of a cancer tumor core, composed of MCF-7, MDA-MB-231 and CSC breast cancer cells, completely surrounded by a stromal compartment composed of human breast fibroblasts or by HBF and THP-1 cells. Bio-inks were produced by combining cells with the decellularized extracellular matrix bioink. FIG. 5A and 6A shows a schematic diagram depicting preparation of the construct. FIG. 5B and 6B,C shows a photograph of the model immediately after bioprinting and crosslinking of the hydrogel.
3. Confocal imaging analysis of 3D constructs was performed in order to assess tissue architecture and relative positions of cell populations. The spatial distribution of each cell population in the 3D model after bioprinting can be observed in FIG. 5C,D, wherein cancer cells core (green) is observed as a rounded structure that is surrounded by HBF (pink). In order to assess construct to construct reproducibility, confocal images of different tumor core constructs and 3D breast cancer models are presented in FIG.7.
4. Interactions between cancer cells and HBF are depicted in FIG.8. Evolution of THP-1 monocytes towards a Macrophage phenotype is depicted in FIG.9.

### Example 5: Bioprinted 3D tumor stroma model subtypes characterization

### 5.1.) Immunofluorescence assay

For immunofluorescence images, 3D tumor-stroma models were washed twice with PBS, fixed with paraformaldehyde 4% (20 min, RT) and left overnight in 30% (w/v) sucrose solution at 4 °C, prior to embedding them in OCT tissue freezing medium. Frozen blocks were stored at -80 °C until needed. Tissue cryosections (30 µm) were then permeabilized with 0.5% Triton X-100 (20 min, RT), and blocked with 2% BSA + 0.1% Triton X-100 in PBS overnight, followed by a ON incubation with primary antibodies (1:200, 4 °C), washed and incubated with secondary antibodies for 1 h, (1:1000, 4 °C), and DAPI (20 min, RT). Immunofluorescence images were acquired on a Zeiss LSM 880 confocal microscope with Zen software (Zeiss Microscopy) and image analysis was performed using imaged software.

### 5.2) Oncoproteome profiler

Proteome Profiler Human XL Cytokine Array assays (R&D Systems, ARY022B) were performed according to the manufacturer's protocol. In brief, culture supernatants from cross-linked dECM, and two biological replicates of bioprinted 3D tumor, stroma, and tumor-stroma models, were collected and added to the array membranes, incubating overnight at 4 °C. Membranes were washed, incubated with a streptavidin-HRP-coupled antibody (1:2000) for 30 min at RT and developed using Chemi-Reagent Mix. Images were captured and visualized using the LI-COR Odyssey Fc imaging system and dot integrated densities were measured using imaged software. Integrated density was calculated as the product of the area of a selected region of interest (ROI) and its mean pixel intensity. ROI areas were manually selected by drawing a region around each dot of the membrane. Further, each sample's dots integrated density values were normalized to each membrane's control dots integrated density.

### 5.3) Results

1. To faithfully represent the diversity of breast tumor subtypes and their molecular features, we intentionally selected distinct tumor cell lines with varying aggressiveness levels to form the core of our 3D model. Subsequently, we progressed to the development of various bioprinted tumor-stroma models that incorporated a bioprinted tumor core of MCF-7 epithelial-like cancer cells, MDA-MB-231 TNBC cells and CSCs isolated from the MDA-MB-231 parental cell line.
2. Immunofluorescence assays were carried out to characterize the ability of the constructs to reproduce the biology of each tumor subtype. Immunofluorescence (IF) staining against NANOG and ALDH1A1 revealed a high expression of those markers in the CSC models, while minimal expression was detected in the MDA-MB-231 cell line and no expression was observed in the MCF-7 model (FIG. 10A). Subsequently, we examined the expression of E-Cadherin, TGF-B, and SLUG proteins, which reflect different states of the epithelial-mesenchymal transition (EMT) process. IF staining confirmed the expression of SLUG and TGF-B biomarkers in cells within the CSC tumor core, whereas no signal for these markers was detected in the MCF-7 tumor cell core (FIG 10B). Notably, MDA-MB-231 cells exhibited intermediate levels of SLUG protein, while no expression of these markers was observed in the MCF-7 model. In contrast, MCF-7 tumor core cells displayed high levels of E-Cadherin, revealing their epithelial-like phenotype, while no expression of this epithelial marker was found in the sections from CSC and MDA-MB-231 tumor core cells, in agreement with the mesenchymal nature of TNBC Staining for cell-type specific markers revealed the activation of biomarkers that are pathologically expressed in tumors.
3. Oncoproteome Array Membranes analysis revealed significant differences among the models (FIG10C). Notably, the array unveiled the upregulation of several oncoprotein markers in the CSC model. Noteworthy among them were the expressions of IL-8, Galectin 3, CMFSCF, IL-6, MMP3, and SERPIN. IL-8, prominently upregulated in the CSC model, plays a pivotal role in the breast TME by influencing proinflammatory immune cell recruitment, promoting angiogenesis and tumor cell invasion, regulating CSC populations, and enhancing resistance to therapy. Similarly, granulocyte-macrophage colony-stimulating factor (GM-CSF), which stimulates immune cell proliferation and activation, was upregulated in the 3D CSC tumor-stroma model, mirroring the inflammatory nature of the CSC TME. Additionally, SERPINE1, recognized as a crucial molecule in CSC formation, was also upregulated in the model along with MMP3, a key contributor of cancer progression and metastasis. Lastly, a pronounced upregulation of IL-6 was detected in the CSC model compared to the other conditions. Notably, IL-6 and IL-8 cytokines have been documented to lead the transition of tumor cells towards CSC-like states. Collectively, these findings underscore the ability of our model to faithfully reproduce the aggressiveness and intrinsic biology of CSC in 3D *in vitro* conditions.

### Example 6: Modelling breast cancer subtypes metastatic potential through the dECM

It was next aimed to determine whether the characterized tumor-stroma models could accurately replicate distinct functional properties such as phenotypic varying metastatic potentials observed across different tumor subtypes. Metastatic potential is a critical factor influencing tumor progression and clinical outcomes in breast cancer patients. Notably, different tumor subtypes exhibit variations in metastatic potential, with hormone receptor-positive tumors of epithelial-like morphology typically demonstrating lower metastatic potential, while TNBC tumors are commonly associated with a highly metastatic nature.

Traditionally, spheroid models have been regarded as gold standards for evaluating invasive potential in 3D cultures. To initially confirm differences in invasive potential among the three cell lines, each cell line was integrated with HBF in a 3D spheroid model setup to assess cancer cells invasion. After 24 hours, the formed spheroids were embedded in dECM, and confocal imaging was conducted to evaluate the invasive potential of each cell type. The resulting images revealed that, after 48 hours, only HBF invaded the surrounding dECM, with no migrating MCF7 cells observed. Conversely, both MDA-MB-231 and CSCs exhibited pronounced invasion toward the dECM, indicating their increased metastatic capacity relative to the MCF-7 cell line. Notably, CSCs demonstrated exceptional migration capabilities toward the dECM, emphasizing their aggressive phenotype (FIG.11A). These results align with previous findings that demonstrated higher invasive capacity for CSCs in 2D invasion assays.

It was next aimed to reproduce those findings into the bioprinted tumor-stroma model framework according to the present invention. For this, MCF-7, MDA-MB-231, and CSC tumor-stroma models were bioprinted and confocal imaging was conducted over time to monitor the behaviour of the cancer cells comprising the tumor cores. As anticipated, the MCF7 tumor model displayed no migration towards the matrix, whereas both the MDA-MB-231 and CSC models exhibited distinct invasion toward the stromal compartment at day 5 compared to day 0 (FIG.11B). These findings affirm that our 3D model successfully emulates the metastatic potential of diverse tumor subtypes, providing a valuable platform for investigating tumor cell metastatic potential towards tumor stroma in a configuration closely resembling in this case breast carcinomas. Moreover, these models offer a robust platform for testing therapeutic agents designed to disrupt tumor-stroma interactions facilitating tumor invasion, as well as evaluating drugs aimed at impeding cancer cell invasiveness.

### Example 7: Modeling Drug Responses in Human Breast Cancer Tumor Models with Stromal Components

### 7.1) Drug cytotoxicity assays

Doxorubicin, Paclitaxel, Docetaxel and Reparixin were purchased from Merck (Original stocks: Docetaxel 1.2 mM and Reparixin 3.5 mM in DMSO, Paclitaxel 1.17 mM in milli-Q water, and Doxorubicin 1.8 mM in milli-Q water). CSC tumor-stroma models according to the invention were sequentially bioprinted in a 96-well high-throughput testing plate **(****FIG.12****)** and then exposed to increasing concentrations of Docetaxel, Paclitaxel or Doxorubicin in monoregimen or in combination with Reparixin for 72 h. Control groups were non-treated cells cultured with DMSO vehicle. Cell media was collected, and drug cytotoxicity was measured using the LDH assay according to manufacturer instructions. Data shown in graphs are the summary of a minimum of three biological replicates in three independent experiments. LDH assays data were normalized to negative (control) and positive (Triton-X 100) cell death controls. Resulting dose-response curves were calculated using GraphPad Prism software through non-linear regression analysis.

### 7.2) Results

A pivotal aspect within breast clinics pertains to the emergence of tumor chemoresistance post-therapy. This phenomenon is associated with the proliferation of CSC clones, which demonstrate resistance to traditional chemotherapy, consequently contributing to tumor recurrence. Noteworthy, our previous research findings underscore the efficacy of the 3D tumor-stroma CSC models to effectively replicate scenarios characterized by the predominance of CSC populations, serving as a valuable platform the evaluation of therapeutic strategies specifically targeted against breast CSC populations.

Identifying and targeting intrinsic biological pathways of CSC holds promise for their eradication. Among the defined targeted therapies in clinical practice, Reparixin emerges as a promising candidate, disrupting the crosstalk between IL-8 and its receptor CXCR1. Remarkably, previous results demonstrated that the CSC 3D tumor-stroma model yielded high levels of IL-8 interleukin, making it a valuable platform for testing the use of this targeted therapy.

Operating on the premise that not only conventional chemotherapy, but also CSC targeted therapy in combination with it, will lead to tumor eradication (FIG.13A), we postulated that Reparixin, when combined with chemotherapy, would exert a higher cytotoxic effect on CSC tumor models.

Considering these factors, it was envisaged to conduct a comprehensive drug screening test. The goal was to assess the effectiveness of various chemotherapeutic compounds in combination with CSC targeted therapy. The aim was to identify the most optimal therapeutic regime for eradicating CSC populations in an in vitro 3D model setup. For this, 96 CSC breast tumor-stroma models were bioprinted in a high-throughput plate (FIG.12). Following 3 days in culture, different therapeutic strategies were applied to the tumor-stroma models, including Paclitaxel, Docetaxel, and Doxorubicin (prominent chemotherapeutic compounds in breast cancer) at increasing concentrations (5, 10, 20, and 50 uM) either in monotherapy or in combination with 50 uM of Reparixin. Concurrently, Reparixin targeted therapy was tested alone at concentrations of 10, 20, 50, and 100 uM. After 72 hours of treatment, cytotoxicity tests were performed to study drug efficacy. Cytotoxicity tests results revealed that Paclitaxel, Docetaxel, and Doxorubicin, in monotherapy regimens, displayed low cytotoxic effects in CSC bioprinted tissue models (FIG.13B). Although CSC models exhibited a positive correlation between drug concentration and cell cytotoxicity, the cytotoxicity remained relatively low at the highest concentrations (50 µM), reaching only the 30% of cell cytotoxicity. In this context, Paclitaxel emerged as the most effective drug candidate, exhibiting the highest cytotoxicity. Additionally, we confirmed that Reparixin targeted therapy exerted high cytotoxic effects at the highest concentrations in monotherapy conditions (50% cell cytotoxicity at 100 µM) (FIG.13C). However, it was decided to fix the Reparixin concentration to 50 µM for the drug combination tests. When testing the combination of chemotherapy plus Reparixin targeted therapy, it was observed that only Paclitaxel in combination with Reparixin achieved a high cytotoxicity in the model (up to 60%), while no prominent effect was observed for Docetaxel and Doxorubicin in combination with Reparixin (FIG.13D). We visualized this cytotoxicity trend using live/dead staining of the CSC model exposed to Paclitaxel in monotherapy or in combination with Reparixin. As shown in FIG.13E, higher Propidium Iodide staining was observed at the outer edge of the core in the models treated with Paclitaxel, suggestive of a significant drug gradient, which may impede successful chemotherapeutic treatment of compact tumors. However, a total staining of the tumor core with PI was observed in the condition of the CSC model treated in combination regimen, highlighting the effectiveness of this therapeutic approach.

Collectively, these results enabled the development of a heatmap guiding the efficacy of each therapy combination, providing a visual and clear indication of which combination therapy approach could yield the most effective treatment against MDA-MB-231 CSC populations (FIG.13F). In this case, a combination of Paclitaxel and Reparixin at 50 µM emerged as the optimal alternative for exerting a significant effect on CSC removal. This type of preclinical drug testing could aid in selecting, based on heatmap visualization, the preferred therapeutic regimen aimed at eradicating CSCs in tumors that ultimately lead to relapse after conventional therapy.

In summary, our findings affirm the capability of various 3D models to serve as a drug screening platform for predicting the effectiveness of a given therapy. This approach holds promise for the efficient screening of numerous compounds in a condensed timeframe, thereby expediting drug discovery in a more cost-effective manner. Additionally, this platform proves instrumental in evaluating the therapeutic efficacy of different compounds against patient-specific tumor cells following therapeutic failure, as patient-derived tumor cells can be readily introduced into the tumor core of the models. These findings thus lay the groundwork for accelerating personalized medicine in a reliable in vitro setup, facilitating both drug discovery and preclinical drug testing.

## Claims

1. A process to prepare a tumor model having an inner core completely surrounded by an outer stromal shell, the process comprising:
a) preparing a supporting bath of a first bioink produced by mixing at least fibroblasts and/or endothelial cells with a dECM in an uncrosslinked state and introducing the mixture in a recipient;
b) preparing a second bioink by mixing at least tumor cells with dECM;
c) introducing an inner core within the supporting bath by bioprinting the second bioink inside the supporting bath prepared in step a) and at a distance of the walls of the recipient to obtain a gel structure formed by the inner core containing the at least tumor cells embedded in dECM completely surrounded in all three dimensions by the outer stromal shell comprising the at least fibroblasts and/or endothelial cells embedded in dECM in an uncrosslinked state; and
d) incubating the gel structure resulting from step c) at a temperature between 25 and 40 °C for the simultaneous crosslinking of the inner core and the outer stromal layer.

2. The process of claim 1, wherein the tumor cells are cells derived from a patient's tumor or selected from the group consisting of cell subtypes derived from breast tumors, pancreatic tumors, lung tumors, kidney tumors, hepatic tumors, prostate tumors, and ovarian tumors.

3. The process of claim 2, wherein the cell subtypes derived from breast tumors are MDA-MB-231, MCF-7 and HCC1806 cell lines; the cell subtypes derived from pancreatic tumors are MIA Paca2 and Capan-2 cell lines; the cell subtypes derived from lung tumors are H69PR and A549 cell lines; the cell subtypes derived from kidney tumors are A-498 and 786-O cell lines; the cell subtypes derived from hepatic tumors are HepG2 and HepT1 cell lines; the cell subtypes derived from prostate tumors are DU145, PC3, and LNCaP cell lines and the cell subtypes derived from ovarian tumors are SK-OV-3, A2780 and OVCAR-3 cell lines.

4. The process according to claims 1 to 3 wherein the dECM is derived from the tissue of origin of the specific designed tumor model, and it is preferably from porcine origin.

5. The process according to any of the previous claims wherein the dECM is derived from a healthy tissue of origin and is absent of tumor-derived factors.

6. The process according to any of the previous claims wherein the first and/or the second bioink may additionally comprise immune cells and the first bioink may comprise other tissue specific stromal cell types including pericytes, adipocytes and/or mesenchymal cells.

7. The process according to any of the previous claims wherein the dECM is devoid of exogenous polymers rheological modifier or crosslinking agent.

8. The process according to claim 1 where the production of the dECM comprises:
i. Dissecting and slicing a tissue of origin;
ii. Washing the tissue resulting from step i) with saline buffer;
iii. Freezing the resulting tissue from step ii);
iv. Treating the resulting tissue from step iii) with 0.5-1% anionic surfactants; and/or bile acids;
v. Washing the resulting tissue from step iv) with water;
vi. Treating the resulting tissue from step v) with nucleases;
vii. Washing the resulting tissue from step vi) with water;
viii. Treating the resulting tissue from step vii) with organic solvents;
ix. Washing the resulting tissue from step viii) with saline buffer;
x. Sterilizing the resulting tissue from step ix);
xi. Freeze-drying and milling the resulting tissue from step x).
xii. Performing an acidic digestion of the powder resulting from step xi).
xiii. Neutralizing the pH of the product resulting from step xii).

9. A tumor model obtainable by a process according to any one of claims 1 to 8.

10. The tumor model according to claim 9 which is a breast tumor model, a pancreatic tumor model, a lung tumor model, a kidney tumor model, a hepatic tumor model, a prostate tumor model or an ovarian tumor model.

11. The tumor model according to any one of claims 9 or 10 which is a metastatic tumor model.

12. The tumor model according to any one of claims 8-10 wherein the inner core has a regular or irregular morphology and wherein the inner core has a maximum diameter in the range of 1 to 6 mm, preferably in the range of 1 to 4 mm and the complete tumor model has a volume in the range of 10 mm³ to 2 cm³, preferably in the range of 6 mm³ to 1 cm³.

13. A method for testing anticancer drugs comprising the step of contacting a drug with a tumor model according to any of claims 9 to 12.

14. The method according to claim 13 which is a high-throughput testing method wherein the testing comprises contacting multiple drugs vis-à-vis with multiple replicas of the tumor model with the same dimensions and morphology, at the same time.

15. The method according to claim 13 which is a high-throughput testing method wherein several tumor models are tested at the same time vis-à-vis a drug.

16. Use of the tumor model according to any of claims 9 to 12 to reproduce the cellular heterogeneity, the spatial architecture and the physiopathological complexity of the tumor microenvironment to develop a personalized medicine and for developing new therapies based on the understanding of the tumor progression in a three-dimensional environment mimicking the tumor microenvironment, preferably of metastatic tumors.
